(19) Europäisches Patentamt
European Patent Office
Office européen des brevets

(11) EP 3 357 961 B1

(12) # EUROPEAN PATENT SPECIFICATION

(45) Date of publication and mention of the grant of the patent:
**05.01.2022 Bulletin 2022/01**

(21) Application number: **16851927.0**

(22) Date of filing: **30.09.2016**

(51) Int Cl.:
***C08L 9/00*** *(2006.01)*
***B60C 1/00*** *(2006.01)*
***C08K 3/00*** *(2018.01)*
***C08K 3/04*** *(2006.01)*
***C08K 5/3477*** *(2006.01)*
***C08L 7/00*** *(2006.01)*
***C08L 9/06*** *(2006.01)*
***C08L 11/00*** *(2006.01)*
***C08L 53/02*** *(2006.01)*
***C07D 257/08*** *(2006.01)*

(86) International application number:
**PCT/JP2016/079170**

(87) International publication number:
**WO 2017/057758 (06.04.2017 Gazette 2017/14)**

(54) **ADDITIVE FOR IMPARTING LOW HEAT BUILD-UP TO RUBBER COMPONENT**

ADDITIV ZUR VERLEIHUNG EINES GERINGEN WÄRMEAUFBAUS AN EINER GUMMIKOMPONENTE

ADDITIF POUR CONFÉRER UNE FAIBLE ACCUMULATION DE CHALEUR À UN COMPOSANT DE CAOUTCHOUC

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.09.2015 JP 2015195368**

(43) Date of publication of application:
**08.08.2018 Bulletin 2018/32**

(73) Proprietor: **Otsuka Chemical Co., Ltd.**
**Osaka-shi, Osaka 540-0021 (JP)**

(72) Inventors:
- **SATO, Takashi**
  **Tokushima-shi**
  **Tokushima 771-0193 (JP)**
- **YUASA, Hiroaki**
  **Tokushima-shi**
  **Tokushima 771-0193 (JP)**
- **NAKASHIMA, Shinya**
  **Tokushima-shi**
  **Tokushima 771-0193 (JP)**

(74) Representative: **Vossius & Partner**
**Patentanwälte Rechtsanwälte mbB**
**Siebertstrasse 3**
**81675 München (DE)**

(56) References cited:
**WO-A2-2017/027521**
**JP-A- S4 947 439**
**JP-A- 2005 200 641**
**JP-A- 2009 526 649**
**JP-A- 2014 515 776**
**JP-A- 2015 093 928**
**JP-A- 2015 093 928**
**JP-B1- S 471 705**
**US-A1- 2014 113 844**

EP 3 357 961 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Technical Field

The present invention relates to an additive for imparting low heat build-up to a rubber component.

### Background Art

Recent environmental concerns have led to strict international regulations on carbon dioxide emissions, and a highly increased demand for lower fuel consumption in automobiles. While the efficiency of drive systems such as engines, as well as transmission systems, greatly contributes to lower fuel consumption, rolling resistance of tires is also largely involved in lower fuel consumption. For lower fuel consumption in automobiles, reducing rolling resistance is important.

As a method for reducing the rolling resistance of tires, applying a rubber composition with low heat build-up to tires is known. Examples of such rubber compositions with low heat build-up include (1) a rubber composition comprising a functionalized polymer having increased affinity to carbon black and silica as fillers (Patent Literature (PTL) 1); (2) a rubber composition comprising a diene elastomer, an inorganic filler as a reinforcing filler, polysulphurized alkoxysilane as a coupling agent, 1,2-dihydropyridine, and a guanidine derivative (Patent Literature (PTL) 2); (3) a rubber composition comprising a rubber component, an aminopyridine derivative, and an inorganic filler (Patent Literature (PTL) 3); and (4) a rubber composition comprising an end-modified polymer and an inorganic filler (Patent Literature (PTL) 4 and Patent Literature PTL 5).

According to the inventions disclosed in PTL 1 to PTL 5, heat build-up of a rubber composition can be reduced by increasing affinity between a filler and a rubber component. As a result, a tire with low hysteresis loss (rolling resistance) can be obtained.

However, the rubber compositions disclosed in PTL 1 to PTL 5 are insufficient in terms of improving low heat build-up.

With increasing demands for lower fuel consumption of automobiles, the development of tires that have highly excellent low heat build-up has been desired.

PTL 6 relates to an inner liner rubber composition containing relative to 100 parts by mass of a rubber component, 0.1-5.0 parts by mass of a tetrazine compound.

PTL 7 relates to lubricant compositions and hydrocarbon fluids including one or more lubricant base stocks and an effective amount of one more zero SAP antiwear additives and/or corrosion inhibitor additives, wherein the one more antiwear and/or corrosion inhibitor additives include one or more functionalized polyolefins having one or more pyridazine moieties.

### Citation List

#### Patent Literature

PTL 1: JP2003-514079A
PTL 2: JP2003-523472A
PTL 3: JP2013-108004A
PTL 4: JP2000-169631A
PTL 5: JP2005-220323A
PTL6: JP2015-093928 A
PTL 7: US 2014/0113844 A1

### Summary of Invention

#### Technical Problem

An object of the present invention is to provide an additive for imparting low heat build-up to a rubber component.

Another object of the present invention is to provide a rubber composition capable of exhibiting low heat build-up.

Another object of the present invention is to provide a modified polymer capable of imparting low heat build-up.

Another object of the present invention is to provide a tire that has excellent low heat build-up.

Solution to Problem

To achieve the above objects, the present inventors carried out extensive research. As a result, the inventors found that a tetrazine compound can impart low heat build-up to a rubber component. Based on this finding, the inventors continued further research, and have accomplished the present invention.
More specifically, the present invention is as defined in the claims.

Advantageous Effects of Invention

The present invention can provide a use of an additive for imparting low heat build-up to a rubber component for at least one member selected from tread, sidewall, bead area, belt, carcass, and shoulder portions of a tire. The additive contains a tetrazine compound, and causes an inorganic filler and/or carbon black to be dispersed in a rubber component.
The present invention can provide a use of a rubber composition and a modified polymer for imparting low heat build-up to a rubber component for at least one member selected from tread, sidewall, bead area, belt, carcass, and shoulder portions of a tire.
The present invention produces a tire using a rubber composition capable of exhibiting low heat build-up, and can thereby reduce rolling resistance of the tire and lower the heat build-up of the tire, thus providing a fuel-efficient tire. Furthermore, even when a rubber composition highly filled with silica is used, excellent low heat build-up can be exhibited. Therefore, the present invention can provide a fuel-efficient tire with high kinematic performance.

Brief Description of Drawings

Fig. 1 shows a $^{13}C$-NMR chart of a tetrazine compound (1b).
Fig. 2 is a $^{13}C$-NMR chart of S-SBR.
Fig. 3 is an enlarged view of Fig. 2.
Fig. 4 is a $^{13}C$-NMR chart of modified S-SBR obtained by modification with the tetrazine compound (1b).
Fig. 5 is an enlarged view of Fig. 4.
Fig. 6 is a diagram for comparing $^{13}C$-NMR charts of the tetrazine compound (1b), S-SBR, and modified S-SBR.

Description of Embodiments

The present invention is described in detail below.

1. Additive for imparting low heat build-up to a rubber component

The additive used for imparting low heat build-up to a rubber component of the present invention (hereinafter sometimes referred to as the "additive of the present invention") includes compounds represented by Formula (1) and salts thereof (hereinafter sometimes referred to as "the tetrazine compound (1)") .

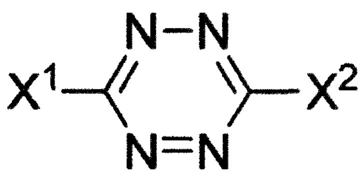
(1)

(wherein $X^1$ and $X^2$ are the same or different and represent a hydrogen atom, or an alkyl, alkylthio, aralkyl, aryl, arylthio, heterocyclic, or amino group, and each of these groups may have one or more substituents).
The "alkyl" as used herein is not particularly limited. Examples include linear, branched, or cyclic alkyl groups. Specific examples include $C_{1-6}$ (particularly $C_{1-4}$) linear or branched alkyl groups, such as methyl, ethyl, n-propyl, isopropyl, n-butyl, isobutyl, s-butyl, t-butyl, 1-ethylpropyl, n-pentyl, neopentyl, n-hexyl, isohexyl, and 3-methylpentyl; $C_{3-8}$ (particularly $C_{3-6}$) cyclic alkyl groups, such as cyclopropyl, cyclobutyl, cyclopentyl, cyclohexyl, cycloheptyl, and cyclooctyl; and the like. The alkyl group is preferably a $C_{1-6}$ linear or branched alkyl group, more preferably methyl, ethyl, n-propyl, isopropyl, n-butyl, isobutyl, or n-pentyl, and particularly preferably methyl or ethyl.
The "alkylthio" as used herein is not particularly limited. Examples include linear, branched, or cyclic alkylthio

groups. Specific examples include $C_{1-6}$ (particularly $C_{1-4}$) linear or branched alkylthio groups, such as methylthio, ethylthio, n-propylthio, isopropylthio, n-butylthio, isobutylthio, s-butylthio, t-butylthio, 1-ethylpropylthio, n-pentylthio, neopentylthio, n-hexylthio, isohexylthio, and 3-methylpentylthio; $C_{3-8}$ (particularly $C_{3-6}$) cyclic alkylthio groups, such as cyclopropylthio, cyclobutylthio, cyclopentylthio, cyclohexylthio, cycloheptylthio, and cyclooctylthio; and the like. The alkylthio group is preferably methylthio, ethylthio, isopropylthio, or isobutylthio, and more preferably methylthio or ethylthio.

The "aralkyl" as used herein is not particularly limited. Examples include benzyl, phenethyl, trityl, 1-naphthylmethyl, 2-(1-naphthyl)ethyl, 2-(2-naphthyl)ethyl, and the like. The aralkyl group is preferably benzyl or phenethyl, and more preferably benzyl.

The "aryl" as used herein is not particularly limited. Examples include phenyl, biphenyl, naphthyl, dihydroindenyl, 9H-fluorenyl, and the like. The aryl group is preferably phenyl or naphthyl, and more preferably phenyl.

The "arylthio" as used herein is not particularly limited. Examples include phenylthio, biphenylthio, naphthylthio, and the like.

The "heterocyclic group" as used herein is not particularly limited. Examples include 2-pyridyl, 3-pyridyl, 4-pyridyl, 2-pyrazinyl, 2-pyrimidyl, 4-pyrimidyl, 5-pyrimidyl, 3-pyridazyl, 4-pyridazyl, 4-(1,2,3-triazyl), 5-(1,2,3-triazyl), 2-(1,3,5-triazyl), 3-(1,2,4-triazyl), 5-(1,2,4-triazyl), 6-(1,2,4-triazyl), 2-quinolyl, 3-quinolyl, 4-quinolyl, 5-quinolyl, 6-quinolyl, 7-quinolyl, 8-quinolyl, 1-isoquinolyl, 3-isoquinolyl, 4-isoquinolyl, 5-isoquinolyl, 6-isoquinolyl, 7-isoquinolyl, 8-isoquinolyl, 2-quinoxalyl, 3-quinoxalyl, 5-quinoxalyl, 6-quinoxalyl, 7-quinoxalyl, 8-quinoxalyl, 3-cinnolyl, 4-cinnolyl, 5-cinnolyl, 6-cinnolyl, 7-cinnolyl, 8-cinnolyl, 2-quinazolyl, 4-quinazolyl, 5-quinazolyl, 6-quinazolyl, 7-quinazolyl, 8-quinazolyl, 1-phthalazyl, 4-phthalazyl, 5-phthalazyl, 6-phthalazyl, 7-phthalazyl, 8-phthalazyl, 1-tetrahydroquinolyl, 2-tetrahydroquinolyl, 3-tetrahydroquinolyl, 4-tetrahydroquinolyl, 5-tetrahydroquinolyl, 6-tetrahydroquinolyl, 7-tetrahydroquinolyl, 8-tetrahydroquinolyl, 1-pyrrolyl, 2-pyrrolyl, 3-pyrrolyl, 2-furyl, 3-furyl, 2-thienyl, 3-thienyl, 1-imidazolyl, 2-imidazolyl, 4-imidazolyl, 5-imidazolyl, 1-pyrazolyl, 3-pyrazolyl, 4-pyrazolyl, 5-pyrazolyl, 2-oxazolyl, 4-oxazolyl, 5-oxazolyl, 2-thiazolyl, 4-thiazolyl, 5-thiazolyl, 3-isoxazolyl, 4-isoxazolyl, 5-isoxazolyl, 3-isothiazolyl, 4-isothiazolyl, 5-isothiazolyl, 4-(1,2,3-thiadiazolyl), 5-(1,2,3-thiadiazolyl), 3-(1,2,5-thiadiazole), 2-(1,3,4-thiadiazole), 4-(1,2,3-oxadiazolyl), 5-(1,2,3-oxadiazolyl), 3-(1,2,4-oxadiazolyl), 5-(1,2,4-oxadiazolyl), 3-(1,2,5-oxadiazolyl), 2-(1,3,4-oxadiazolyl), 1-(1,2,3-triazolyl), 4-(1,2,3-triazolyl), 5-(1,2,3-triazolyl), 1-(1,2,4-triazolyl), 3-(1,2,4-triazolyl), 5-(1,2,4-triazolyl), 1-tetrazolyl, 5-tetrazolyl, 1-indolyl, 2-indolyl, 3-indolyl, 4-indolyl, 5-indolyl, 6-indolyl, 7-indolyl, 1-isoindolyl, 2-isoindolyl, 3-isoindolyl, 4-isoindolyl, 5-isoindolyl, 6-isoindolyl, 7-isoindolyl, 1-benzimidazolyl, 2-benzimidazolyl, 4-benzimidazolyl, 5-benzimidazolyl, 6-benzimidazolyl, 7-benzimidazolyl, 2-benzofuranyl, 3-benzofuranyl, 4-benzofuranyl, 5-benzofuranyl, 6-benzofuranyl, 7-benzofuranyl, 1-isobenzofuranyl, 3-isobenzofuranyl, 4-isobenzofuranyl, 5-isobenzofuranyl, 6-isobenzofuranyl, 7-isobenzofuranyl, 2-benzothienyl, 3-benzothienyl, 4-benzothienyl, 5-benzothienyl, 6-benzothienyl, 7-benzothienyl, 2-benzoxazolyl, 4-benzoxazolyl, 5-benzoxazolyl, 6-benzoxazolyl, 7-benzoxazolyl, 2-benzothiazolyl, 4-benzothiazolyl, 5-benzothiazolyl, 6-benzothiazolyl, 7-benzothiazolyl,1-indazolyl, 3-indazolyl, 4-indazolyl, 5-indazolyl, 6-indazolyl, 7-indazolyl, 2-morpholyl, 3-morpholyl, 4-morpholyl, 1-piperazyl, 2-piperazyl, 1-piperidyl, 2-piperidyl, 3-piperidyl, 4-piperidyl, 2-tetrahydropyranyl, 3-tetrahydropyranyl, 4-tetrahydropyranyl, 2-tetrahydrothiopyranyl, 3-tetrahydrothiopyranyl, 4-tetrahydrothiopyranyl, 1-pyrrolidyl, 2-pyrrolidyl, 3-pyrrolidyl, 2-tetrahydrofuranyl, 3-tetrahydrofuranyl, 2-tetrahydrothienyl, 3-tetrahydrothienyl, and the like. Among these, the heterocyclic group is preferably pyridyl, furanyl, thienyl, pyrimidyl, or pyrazyl, and is more preferably pyridyl.

The "amino" as used herein includes an amino group represented by $-NH_2$ and substituted amino groups. Examples of substituted amino groups include $C_{1-6}$ (particularly $C_{1-4}$) linear or branched monoalkylamino groups, such as methylamino, ethylamino, n-propylamino, isopropylamino, n-butylamino, isobutylamino, s-butylamino, t-butylamino, 1-ethylpropylamino, n-pentylamino, neopentylamino, n-hexylamino, isohexylamino, and 3-methylpentylamino; and dialkylamino groups having two $C_{1-6}$ (particularly $C_{1-4}$) linear or branched alkyl groups, such as dimethylamino, ethlmethylamino, and diethylamino.

Each of the alkyl, alkylthio, aralkyl, aryl, arylthio, heterocyclic, and amino groups may have one or more substituents. The "substituent" is not particularly limited. Examples of substituents include halogen atoms and amino, aminoalkyl, alkoxycarbonyl, acyl, acyloxy, amide, carboxyl, carboxyalkyl, formyl, nitrile, nitro, alkyl, hydroxyalkyl, hydroxy, alkoxy, aryl, aryloxy, heterocyclic, thiol, alkylthio, arylthio, and like groups. The number of substituents is preferably 1 to 5, and more preferably 1 to 3.

The "halogen atom" as used herein includes fluorine, chlorine, bromine, and iodine atoms. Preferable halogen atoms are chlorine, bromine, and iodine atoms.

The "aminoalkyl" as used herein is not particularly limited. Examples include aminoalkyl groups, such as aminomethyl, 2-aminoethyl, and 3-aminopropyl.

The "alkoxycarbonyl" as used herein is not particularly limited. Examples include methoxycarbonyl, ethoxycarbonyl, and the like.

The "acyl" as used herein is not particularly limited. Examples include $C_{1-4}$ linear or branched alkylcarbonyl groups, such as acetyl, propionyl, and pivaloyl.

The "acyloxy" as used herein is not particularly limited. Examples include acetyloxy, propionyloxy, n-butyryloxy, and the like.

The "amide" as used herein is not particularly limited. Examples include carboxylic acid amide groups, such as acetamide and benzamide; thioamides such as thioacetamide and thiobenzamide; N-substituted amides such as N-methylacetamide and N-benzylacetamide; and the like.

The "carboxyalkyl" as used herein is not particularly limited. Examples include carboxy-alkyl groups (preferably carboxy-containing alkyl groups having 1 to 6 carbon atoms), such as carboxymethyl, carboxyethyl, carboxy-n-propyl, carboxy-n-butyl, carboxy-n-butyl, and carboxy-n-hexyl.

The "hydroxyalkyl" as used herein is not particularly limited. Examples include hydroxyalkyl groups (hydroxy-containing alkyl groups having 1 to 6 carbon atoms), such as hydroxymethyl, hydroxyethyl, hydroxy-n-propyl, and hydroxy-n-butyl.

The "alkoxy" as used herein is not particularly limited. Examples include linear, branched, or cyclic alkoxy groups. Specific examples include $C_{1-6}$ (particularly $C_{1-4}$) linear or branched alkoxy groups, such as methoxy, ethoxy, n-propoxy, isopropoxy, n-butoxy, t-butoxy, n-pentyloxy, neopentyloxy, and n-hexyloxy; $C_{3-8}$ (particularly $C_{3-6}$) cyclic alkoxy groups, such as cyclopropyloxy, cyclobutyloxy, cyclopenthyloxy, cyclohexyloxy, cycloheptyloxy, and cyclooctyloxy; and the like.

The "aryloxy" as used herein is not particularly limited. Examples include phenoxy, biphenyloxy, naphthoxy, and the like.

The "salt" of the tetrazine compound represented by Formula (1) is not particularly limited and includes all types of salts. Examples of such salts include inorganic acid salts such as hydrochloride, sulfate, and nitrate; organic acid salts such as acetate and methanesulfonate; alkali metal salts such as sodium salt and potassium salt; alkaline earth metal salts such as magnesium salt and calcium salt; quaternary ammonium salts such as dimethyl ammonium and triethyl ammonium; and the like.

Of these tetrazine compounds (1), preferable compounds are those wherein $X^1$ and $X^2$ are the same or different and represent an optionally substituted alkyl group, an optionally substituted aralkyl group, an optionally substituted aryl group, or an optionally substituted heterocyclic group.

More preferable tetrazine compounds (1) are those wherein $X^1$ and $X^2$ are the same or different and represent an optionally substituted aralkyl group, an optionally substituted aryl group, or an optionally substituted heterocyclic group.

Still more preferable tetrazine compounds (1) are those wherein $X^1$ and $X^2$ are the same or different and represent an optionally substituted benzyl group, an optionally substituted phenyl group, an optionally substituted 2-pyridyl group, an optionally substituted 3-pyridyl group, an optionally substituted 4-pyridy group, an optionally substituted 2-furanyl group, an optionally substituted thienyl group, an optionally substituted 1-pyrazolyl group, an optionally substituted 2-pyrimidyl group, or an optionally substituted 2-pyrazyl group. Among these, compounds wherein $X^1$ and $X^2$ are the same or different and represent an optionally substituted 2-pyridyl, an optionally substituted 3-pyridyl group, or an optionally substituted 2-furanyl group are particularly preferable.

Specific examples of the tetrazine compound (1) include 1,2,4,5-tetrazine, 3,6-bis(2-pyridyl)-1,2,4,5-tetrazine, 3,6-bis(3-pyridyl)-1,2,4,5-tetrazine, 3,6-bis(4-pyridyl)-1,2,4,5-tetrazine, 3,6-diphenyl-1,2,4,5-tetrazine, 3,6-dibenzyl-1,2,4,5-tetrazine, 3,6-bis(2-furanyl)-1,2,4,5-tetrazine, 3-methyl-6-(3-pyridyl)-1,2,4,5-tetrazine, 3,6-bis(3,5-dimethyl-1-pyrazolyl)-1,2,4,5-tetrazine, 3,6-bis(2-thienyl)-1,2,4,5-tetrazine, 3-methyl-6-(2-pyridyl)-1,2,4,5-tetrazine, 3,6-bis(4-hydroxyphenyl)-1,2,4,5-tetrazine, 3,6-bis(3-hydroxyphenyl)-1,2,4,5-tetrazine, 3,6-bis(2-pyrimidinyl)-1,2,4,5-tetrazine, 3,6-bis(2-pyrazyl)-1,2,4,5-tetrazine, and the like.

Among these, preferable tetrazine compounds (1) are 3,6-bis(2-pyridyl)-1,2,4,5-tetrazine, 3,6-bis(3-pyridyl)-1,2,4,5-tetrazine, 3,6-bis(2-furanyl)-1,2,4,5-tetrazine, 3-methyl-6-(3-pyridyl)-1,2,4,5-tetrazine, and 3-methyl-6-(2-pyridyl)-1,2,4,5-tetrazine. More preferable tetrazine compounds (1) are 3,6-bis(2-pyridyl)-1,2,4,5-tetrazine and 3,6-bis(3-pyridyl)-1,2,4,5-tetrazine.

Adding the tetrazine compound (1) to a rubber component can impart low heat build-up to the rubber component. A tire manufactured (produced) from a rubber composition comprising such a tetrazine compound (1) has low heat build-up, which reduces rolling resistance, thus exhibiting low fuel consumption performance.

Rubber component

The rubber component as used herein is not particularly limited. Examples include natural rubbers (NR), synthetic diene rubbers, and a mixture of natural rubber and synthetic diene rubber; and non-diene rubbers other than these rubbers.

Examples of natural rubbers include natural rubber latex, technically specified rubber (TSR), ribbed smoked sheet (RSS), gutta-percha, *Chinese gulta percha (Eucommia ulmoides)*-derived natural rubber, guayule-derived natural rubber, Russian dandelion (*Taraxacum kok-saghyz*)-derived natural rubber, and the like. Examples of natural rubbers of the present invention further include modified natural rubbers obtained by modifying these rubbers, such as epoxidated natural rubber, methacrylic acid modified natural rubber, and styrene modified natural rubber.

Examples of synthetic diene rubbers include styrenebutadiene copolymer rubber (SBR), butadiene rubber (BR),

isoprene rubber (IR), nitrile rubber (NBR), chloroprene rubber (CR), ethylene-propylene-diene terpolymer rubber (EPDM), styreneisoprene-styrene triblock copolymer (SIS), styrene-butadienestyrene triblock copolymer (SBS), and the like; and modified synthetic diene rubbers thereof. Examples of modified synthetic diene rubbers include main chain-modified, one-terminal-modified, both-terminals-modified, or like modified diene rubbers. Examples of modified functional groups of modified synthetic diene rubbers include various functional groups, such as epoxy, amino, alkoxysilyl, and hydroxy groups. Modified synthetic diene rubbers may contain one or more such functional groups.

The method for producing a synthetic diene rubber is not particularly limited. Examples of production methods include emulsion polymerization, solution polymerization, radical polymerization, anionic polymerization, cationic polymerization, and the like. The glass transition point of the synthetic diene rubber is also not particularly limited.

The cis/trans/vinyl ratio of the double bond portions of natural rubber and synthetic diene rubber is not particularly limited. The rubber at any cis/trans/vinyl ratio can be preferably used. The number average molecular weight and molecular weight distribution of the diene rubber are not particularly limited. The diene rubber preferably has a number average molecular weight of 500 to 3000000, and a molecular weight distribution of 1.5 to 15.

A wide variety of non-diene rubbers can be used as the non-diene rubber.

The rubber component can be used singly, or as a mixture (blend) of two or more. Among these, the rubber component is preferably natural rubber, IR, SBR, BR, or a mixture of two or more of these rubbers. More preferably, the rubber component is natural rubber, SBR, BR, or a mixture of two or more of these rubbers. Although the blending ratio of these rubbers is not particularly limited, SBR, BR, or a mixture thereof is preferably present in an amount of 50 to 100 parts by mass, and particularly preferably 75 to 100 parts by mass, per 100 parts by mass of the rubber component. When a mixture of SBR and BR is incorporated, the total amount of SBR and BR is preferably within the above-mentioned range. In this case, the amount of SBR is preferably in the range of 50 to 100 parts by mass, and the amount of BR is preferably in the range of 0 to 50 parts by mass.

## 2. Modified Polymer

The modified polymer used in the present invention is produced by using a diene rubber and a rubber mixture comprising the additive of the present invention.

That is, the modified polymer used in the present invention is obtained by treating a diene rubber using the tetrazine compound (1).

By allowing the tetrazine compound (1) to act on a diene rubber modified with an epoxy, amino, alkoxysilyl, hydroxy, or like group, a further modified rubber can be obtained.

The raw materials for producing the modified polymer used in the present invention include the tetrazine compound (1) and a diene rubber. The amount of the tetrazine compound (1) is not particularly limited. The amount may be appropriately adjusted, for example, so that the tetrazine compound (1) is generally present in an amount of 0.1 to 10 parts by mass, preferably 0.2 to 5 parts by mass, and more preferably 0.5 to 2 parts by mass, per 100 parts by mass of the rubber component of the rubber composition described below.

The modified polymer used in the present invention contains a heteroatom, such as a nitrogen atom. This heteroatom interacts strongly with silica and carbon black, and thus enhances the dispersibility of silica or carbon black in a diene rubber component, thus imparting excellent low heat build-up to the modified polymer.

The modified polymer used in the present invention preferably has at least one of the compound structures represented by the following formulas (2) to (12):

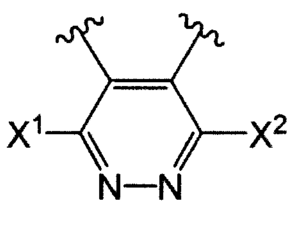 (2)、 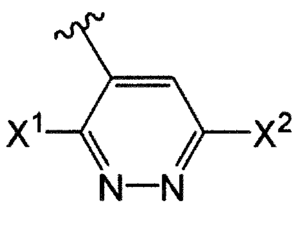 (3)、 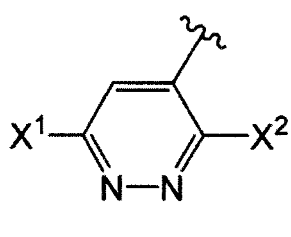 (4)、

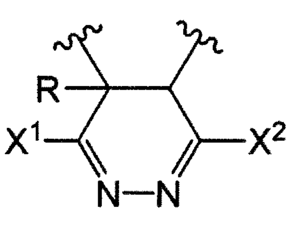 (5)、 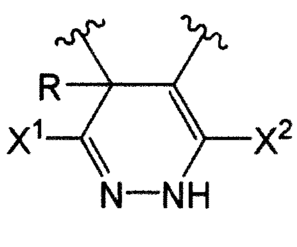 (6)、 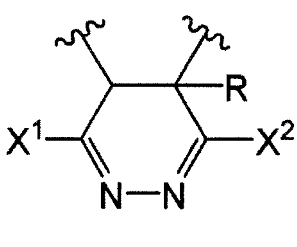 (7)、

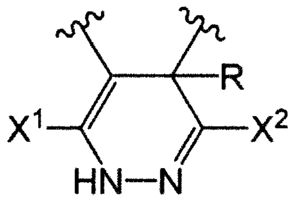 (8)、 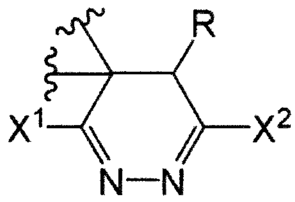 (9)、 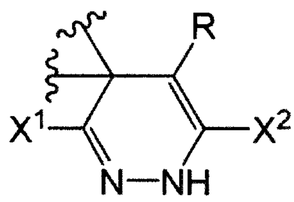 (10)、

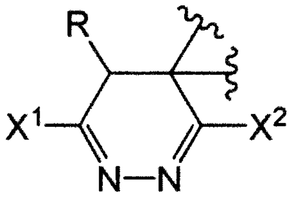 (11)、 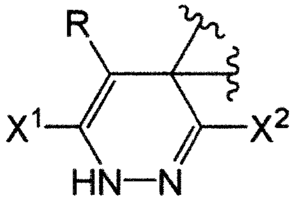 (12)

(wherein $X^1$ and $X^2$ are the same as defined in Item 1, and R represents a halogen atom or alkyl).

The modified polymer used in the present invention is considered to be obtained by the following reaction mechanism.

Reaction mechanism of the rubber component with the additive of the present invention

An inverse electron-demand Aza-Diels-Alder reaction proceeds between the tetrazine compound (1) and double bonds in the rubber component.

Specifically, when the reactions as shown in the following Reaction Scheme-1 to Reaction Scheme-4 proceed, the tetrazine compound (1) is bound to double bond sites of a diene rubber to form six-membered ring structures, thus forming a modified polymer.

Reaction Scheme-1

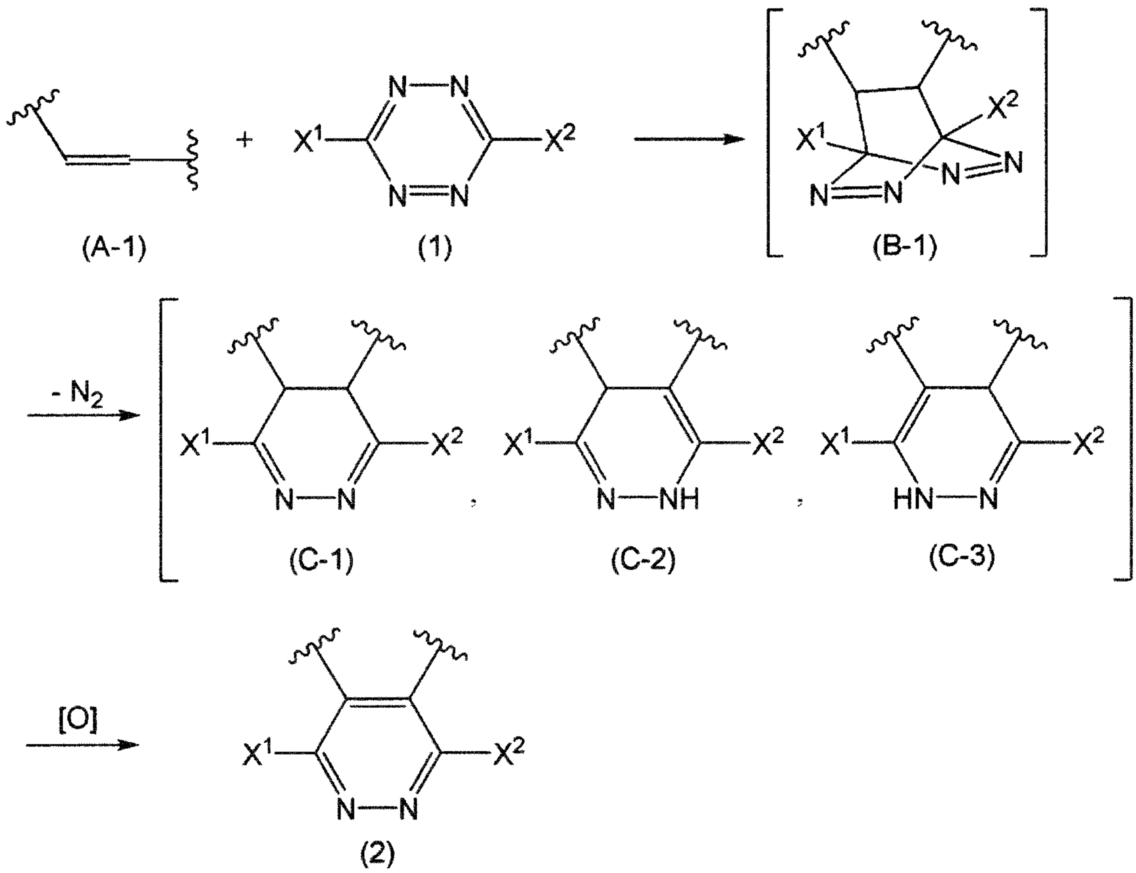


(A-1) (1) (B-1)

(C-1), (C-2), (C-3)

(2)

(wherein $X^1$ and $X^2$ are as defined above) .

In Reaction Scheme-1, the inverse electron-demand Aza-Diels-Alder reaction between the double bond sites of a diene rubber represented by formula (A-1) and the tetrazine compound (1) forms bicyclic ring structures represented by formula (B-1). Denitrogenation in the -N=N-portions of the bicyclic ring structure easily proceeds to form six-membered ring structures represented by the formulas (C-1), (C-2), and/or (C-3). The obtained structures are further oxidized with oxygen in the air to produce a modified polymer having six-membered ring structures represented by formula (2).

Reaction Scheme-2

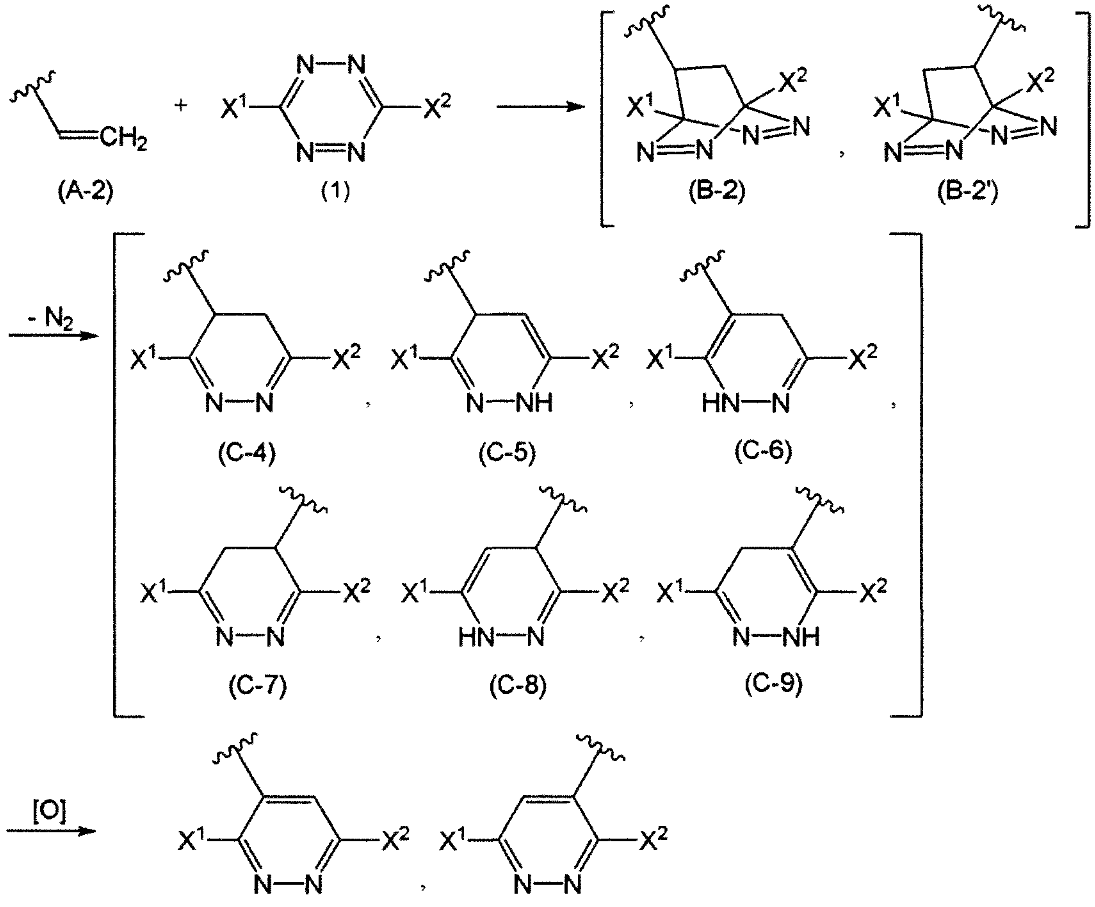


(A-2) (1) (B-2) , (B-2')

(C-4) , (C-5) , (C-6) ,

(C-7) , (C-8) , (C-9)

(3) , (4)

(wherein $X^1$ and $X^2$ are as defined above) .

In Reaction Scheme-2, as in Reaction Scheme-1, the reaction between the double bond sites of a diene rubber represented by formula (A-2) and the tetrazine compound (1) forms bicyclic ring structures represented by formulas (B-2) and/or (B-2'). After six-membered ring structures represented by formulas (C-4) to (C-9) are then formed, a modified polymer having six-membered structures represented by formulas (3) and/or (4) is produced.

Reaction Scheme-3

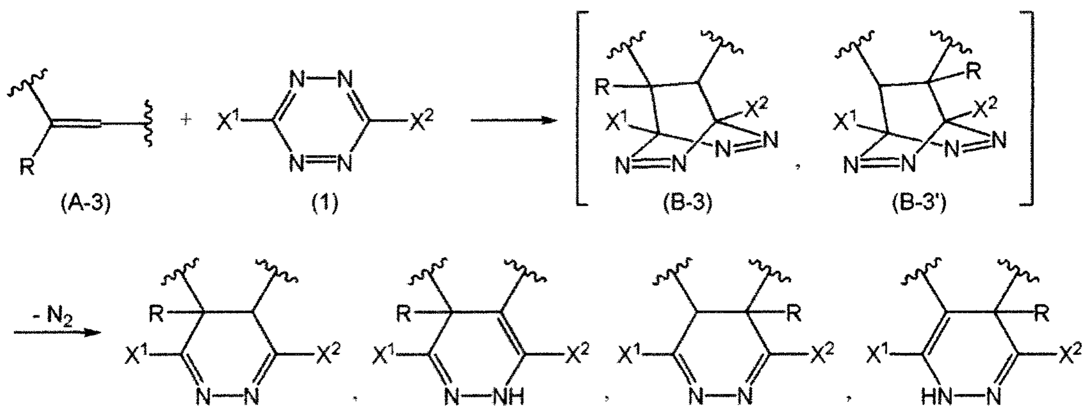


(A-3) (1) (B-3) , (B-3')

(5) , (6) , (7) , (8)

(wherein $X^1$ and $X^2$ are as defined above, and R is alkyl or a halogen atom).

In Reaction Scheme-3, after the inverse electron-demand Aza-Diels-Alder reaction between the double bond sites of a diene rubber represented by formula (A-3) and the tetrazine compound (1) forms bicyclic ring structures represented by formulas (B-3) and/or (B-3'), a nitrogen molecule is released from the structure to produce a modified polymer having six-membered ring structures represented by formulas (5) to (8). Further, when R on the double bond site of the diene rubber represented by formula (A-3) is a halogen atom, the halogen atom may be eliminated. In that case, a modified polymer having six-membered ring structures represented by formula (2) is produced by an oxidation reaction.

Reaction Scheme-4

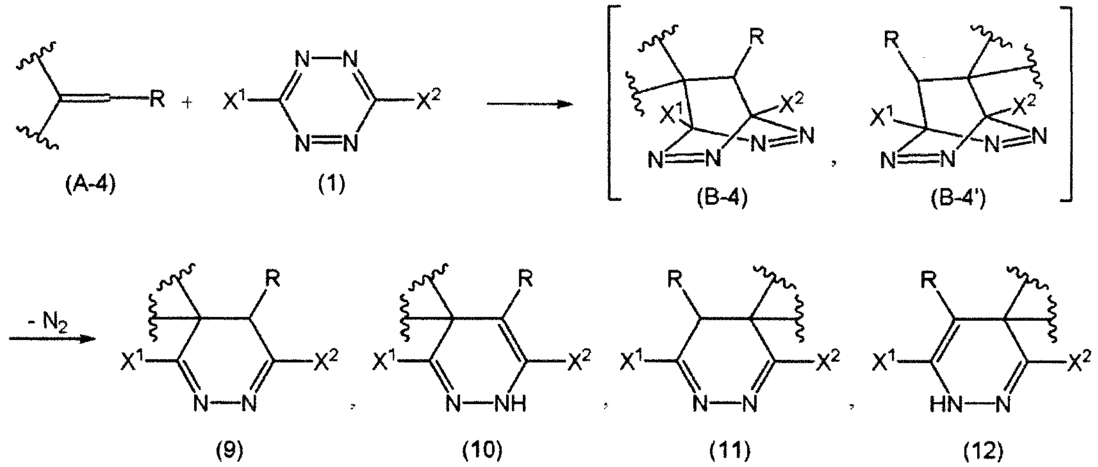


(A-4) (1) (B-4) , (B-4')

(9) , (10) , (11) , (12)

(wherein $X^1$, $X^2$, and R are as defined above) .

In Reaction Scheme-4, as in the reaction shown in Reaction Scheme-3, after the reaction between the double bond sites of a diene rubber represented by formula (A-4) and the tetrazine compound (1) forms bicyclic ring structures represented by formulas (B-4) and/or (B-4'), a modified polymer having six-membered ring structures represented by formulas (9) to (12) is produced.

Further, silica can be dispersed in a rubber component by the action of the additive of the present invention. The silica dispersion mechanism is presumed to be as follows.

Silica dispersion mechanism

The nitrogen atoms in the tetrazine compound (1), which is contained in the additive of the present invention, have high affinity to silica. The modified polymer produced by a reaction between the rubber component and the tetrazine compound (1) is presumed to have improved affinity to silica due to the presence of nitrogen atoms derived from the tetrazine compound. In particular, introduction of a heteroatom-containing substituent or polar group to the 3-position ($X^1$ group) and the 6-position ($X^2$ group) of the tetrazine compound is presumed to increase affinity to silica. The additive of the present invention is thus considered to disperse silica in the rubber component.

Method for producing the modified polymer

The method for producing the modified polymer used in the present invention is not particularly limited. The modified polymer used in the present invention is produced, for example, using a rubber mixture containing at least one rubber component selected from the group consisting of natural rubbers and synthetic diene rubbers, and the tetrazine compound (1).

Specific examples of the method for producing the modified polymer used in the present invention are as follows. When the rubber component is a solid, for example, a method comprising kneading the rubber component with the tetrazine compound (1) under heating conditions (a kneading method) can be used. When the rubber component is in a liquid form (a liquid), for example, a method comprising mixing a solution or emulsion (suspension) of the rubber component and the tetrazine compound (1) under heating conditions (a liquid mixing method) can be used.

The heating temperature is not particularly limited. For example, when the kneading method is used, the upper limit of the temperature of the rubber composition is preferably 80 to 190°C, more preferably 90 to 160°C, and still more

preferably 100 to 150°C. When the liquid mixing method is used, the upper limit of the temperature of the liquid rubber composition is 80 to 190°C, more preferably 90 to 160°C, and still more preferably 100 to 150°C.

The mixing time or kneading time is not particularly limited. For example, when the kneading method is used, the kneading time is preferably 10 seconds to 20 minutes, more preferably 30 seconds to 10 minutes, and still more preferably 60 seconds to 7 minutes. When the liquid mixing method is used, the mixing time is preferably 10 seconds to 60 minutes, more preferably 30 seconds to 40 minutes, and still more preferably 60 seconds to 30 minutes. After the mixing reaction is performed by the liquid mixing method, for example, the solvent is evaporated (removed) from the mixture under reduced pressure, and a solid rubber composition is collected.

In the method for producing the modified polymer used in the present invention, the amount of the tetrazine compound (1) is not particularly limited. For example, the tetrazine compound (1) is usually used in an amount of 0.1 to 10 parts by mass, preferably 0.25 to 5 parts by mass, and more preferably 0.5 to 2 parts by mass, per 100 parts by mass of the rubber component in the rubber composition.

## 3. Rubber Composition

The rubber composition used in the present invention comprises a rubber component, the additive of the present invention, and an inorganic filler and/or carbon black.

The rubber composition used in the present invention comprises the modified polymer and an inorganic filler and/or carbon black.

The rubber component, the additive of the present invention, and the modified polymer are as described above.

The amount of the additive of the present invention is usually 0.1 to 10 parts by mass, preferably 0.25 to 5 parts by mass, and more preferably 0.5 to 2 parts by mass, per 100 parts by mass of the rubber component in the rubber composition.

The amount of the inorganic filler and/or carbon black is not particularly limited. For example, the inorganic filler and/or carbon black is usually 2 to 200 parts by mass, preferably 30 to 130 parts by mass, and more preferably 35 to 110 parts by mass, per 100 parts by mass of the rubber component. When the inorganic filler and carbon black are both used, their amounts are appropriately adjusted so that the total amount of these components falls within the above-mentioned range.

Incorporating 2 parts by mass or more of an inorganic filler and/or carbon black is preferable from the viewpoint of improving the rubber composition reinforcement, whereas incorporating 200 parts by mass or less of an inorganic filler and/or carbon black is preferable from the viewpoint of reducing rolling resistance. When an inorganic filler and/or carbon black is used, a master batch prepared by wet- or dry-mixing the inorganic filler and/or carbon black with the polymer beforehand may be used.

The inorganic filler or carbon black is usually used to enhance reinforcement of the rubber. In this specification, inorganic fillers do not include carbon black.

### Inorganic filler

The inorganic filler is not particularly limited as long as it is an inorganic compound usually used in the rubber industry. Examples of usable inorganic compounds include silica; aluminas ($Al_2O_3$) such as γ-alumina and α-alumina; alumina monohydrates ($Al_2O_3 \cdot H_2O$) such as boehmite and diaspore; aluminum hydroxides [$Al(OH)_3$] such as gibbsite and bayerite; aluminum carbonate [$Al_2(CO_3)_2$], magnesium hydroxide [$Mg(OH)_2$], magnesium oxide (MgO), magnesium carbonate ($MgCO_3$), talc ($3MgO \cdot 4SiO_2 \cdot H_2O$), attapulgite ($5MgO \cdot 8SiO_2 \cdot 9H_2O$), titanium white ($TiO_2$), titanium black ($TiO_{2n-1}$), calcium oxide (CaO), calcium hydroxide [$Ca(OH)_2$], magnesium aluminum oxide ($MgO \cdot Al_2O_3$), clay ($Al_2O_3 \cdot 2SiO_2$), kaolin ($Al_2O_3 \cdot 2SiO_2 \cdot 2H_2O$), pyrophyllite ($Al_2O_3 \cdot 4SiO_2 \cdot H_2O$), bentonite ($Al_2O_3 \cdot 4SiO_2 \cdot 2H_2O$), aluminium silicates ($Al_2SiO_5$, $Al_4 \cdot 3SiO_4 \cdot 5H_2O$, etc.), magnesium silicates ($Mg_2SiO_4$, $MgSiO_3$, etc.), calcium silicate ($Ca_2 \cdot SiO_4$, etc.), aluminum calcium silicates ($Al_2O_3 \cdot CaO \cdot 2SiO_2$, etc.), magnesium calcium silicate ($CaMgSiO_4$), calcium carbonate ($CaCO_3$), zirconium oxide ($ZrO_2$), zirconium hydroxide [$ZrO(OH)_2 \cdot nH_2O$], zirconium carbonate [$Zr(CO_3)_2$], zinc acrylate, zinc methacrylate, and crystalline aluminosilicates containing hydrogen, alkali metal, or alkaline earth metal that compensate charge, such as various types of zeolites. To enhance affinity to the rubber component, the surface of these inorganic fillers may be treated with an organic compound.

The amount of the inorganic filler is usually 10 to 200 parts by mass per 100 parts by mass of the rubber component.

From the viewpoint of imparting rubber strength, silica is preferably used as the inorganic filler. Using silica alone or a combination of silica with one or more inorganic compounds usually used in the rubber industry is more preferable. When the inorganic filler is a combination of silica with one or more inorganic compounds other than silica, their amounts may be appropriately adjusted so that the total amount of the inorganic filler components falls within the above-mentioned range.

Adding silica is preferable because it can impart rubber strength. As silica, any type of commercially available products can be used. Among these, wet silica, dry silica, or colloidal silica is preferable, and wet silica is more preferable. To enhance affinity to the rubber component, the surface of silica may be treated with an organic compound.

The BET specific surface area of silica is not particularly limited and may be, for example, in the range of 40 to 350 $m^2/g$. Silica that has a BET specific surface area within this range is advantageous in that rubber reinforcement and dispersibility in the rubber component can both be achieved. The BET specific surface area is measured according to ISO 5794/1.

Silica preferable from this viewpoint is silica having a BET specific surface area of 80 to 300 $m^2/g$, more preferably silica having a BET specific surface area of 100 to 270 $m^2/g$, and particularly preferably a silica having a BET specific surface area of 110 to 270 $m^2/g$.

Examples of commercially available products of such silica include products under the trade names of: "HD165MP" (BET specific surface area: 165 $m^2/g$), "HD115MP" (BET specific surface area: 115 $m^2/g$), "HD200MP" (BET specific surface area: 200 $m^2/g$), and "HD250MP" (BET specific surface area: 250 $m^2/g$), all produced by Quechen Silicon Chemical Co., Ltd.; "Nipsil AQ" (BET specific surface area: 205 $m^2/g$) and "Nipsil KQ" (BET specific surface area: 240 $m^2/g$), both produced by Tosoh Silica Corporation; "Ultrasil VN3" (BET specific surface area: 175 $m^2/g$) produced by Degussa AG; and the like.

The amount of silica is usually 20 to 120 parts by mass, preferably 30 to 100 parts by mass, and more preferably 40 to 90 parts by mass, per 100 parts by mass of the rubber component.

Although adding silica usually improves kinematic performance, adding a large amount of silica tends to deteriorate low heat build-up. However, when the additive of the present invention is used, excellent low heat build-up can be exhibited even when a large amount of silica is incorporated.

In particular, to achieve both kinematic performance and low fuel performance, the amount of silica is usually 40 to 120 parts by mass, preferably 60 to 115 parts by mass, and more preferably 70 to 110 parts by mass, per 100 parts by mass of the rubber component.

When the tetrazine compound (1) is incorporated into a rubber composition containing an inorganic filler, in particular, silica, dispersibility of silica can be significantly improved, thus remarkably improving low heat build-up of the rubber composition. Specifically, the additive of the present invention can be used as a dispersant for inorganic fillers and/or carbon black, a heat build-up reducer, a heat build-up inhibitor, or a heat build-up suppressor. Preferably, the additive of the present invention can be used as a dispersant for rubbers, a heat build-up reducer for rubbers, a heat build-up inhibitor for rubbers, or a heat build-up suppressor for rubbers.

Carbon black

The carbon black for use is not particularly limited. For example, commercially available carbon blacks, carbon-silica dual phase fillers, and the like can be used. Incorporating carbon black to a rubber component reduces electric resistance of rubber, thus providing an electric charge-suppressing effect and a rubber strength-enhancing effect.

Specific examples of carbon blacks include high, middle or low-structure SAF, ISAF, IISAF, N110, N134, N220, N234, N330, N339, N375, N550, HAF, FEF, GPF, or SRF-grade carbon black, and the like. Among these, SAF, ISAF, IISAF, N134, N234, N330, N339, N375, HAF, or FEF-grade carbon black is preferable.

There is no particular limitation on the DBP absorption of the carbon black. The carbon black preferably have a DBP absorption of 60 to 200 $cm^3/100$ g, more preferably 70 to 180 $cm^3/100$ g, and particularly preferably 80 to 160 $cm^3/100$ g.

The carbon black preferably has a nitrogen adsorption specific surface area (N2SA, measured according to JIS K6217-2: 2001) of 30 to 200 $m^2/g$, more preferably 40 to 180 $m^2/g$, particularly preferably 50 to 160 $m^2/g$.

In the rubber composition containing carbon black, the tetrazine compound (1) or a reaction product of the rubber component and tetrazine compound (1) is believed to strongly interact with carbon black. Therefore, when the rubber composition of the present invention is used, dispersibility of carbon black, in particular, is increased significantly, and low heat build-up of the rubber composition can be significantly improved.

The amount of carbon black is usually 2 to 150 parts by mass, preferably 4 to 120 parts by mass, and more preferably 6 to 100 parts by mass, per 100 parts by mass of the rubber component.

Two parts by mass or more of carbon black is preferable in terms of securing antistatic performance and rubber strength performance, whereas 150 parts by mass or less of carbon black is preferable in terms of reducing rolling resistance.

Other ingredients

The rubber composition used in the present invention may contain, in addition to the tetrazine compound (1) and an inorganic filler and/or carbon black, ingredients usually used in the rubber industry. Such ingredients can be

appropriately selected from, for example, antioxidants, ozone protectants, softeners, processing aids, waxes, resins, foaming agents, oils, stearic acid, zinc oxide (ZnO), vulcanization accelerators, vulcanization retarders, vulcanizing agents (sulfur), and the like, as long as the ingredients do not impair the object of the present invention. As such ingredients, commercially available products can be preferably used.

Further, a silane coupling agent may be incorporated into the rubber composition comprising an inorganic filler, such as silica, for the purpose of enhancing the rubber composition reinforcement by silica, or enhancing wear resistance and low heat build-up of the rubber composition.

The silane coupling agent that can be used with an inorganic filler is not particularly limited, and commercially available products can be preferably used. Examples of such silane coupling agents include sulfide, polysulfide, thioester, thiol, olefin, epoxy, amino, or alkyl silane coupling agents.

Examples of sulfide silane coupling agents include bis(3-triethoxysilylpropyl)tetrasulfide, bis(3-trimethoxysilylpropyl)tetrasulfide, bis(3-methyldimethoxysilylpropyl)tetrasulfide, bis(2-triethoxysilylethyl)tetrasulfide, bis(3-triethoxysilylpropyl)disulfide, bis(3-trimethoxysilylpropyl)disulfide, bis(3-methyldimethoxysilylpropyl)disulfide, bis(2-triethoxysilylethyl)disulfide, bis(3-triethoxysilylpropyl)trisulfide, bis(3-trimethoxysilylpropyl)trisulfide, bis(3-methyldimethoxysilylpropyl)trisulfide, bis(2-triethoxysilylethyl)trisulfide, bis(3-monoethoxydimethylsilylpropyl)tetrasulfide, bis(3-monoethoxydimethylsilylpropyl)trisulfide, bis(3-monoethoxydimethylsilylpropyl)disulfide, bis(3-monomethoxydimethylsilylpropyl)tetrasulfide, bis(3-monomethoxydimethylsilylpropyl)trisulfide, bis(3-monomethoxydimethylsilylpropyl)disulfide, bis(2-monoethoxydimethylsilylethyl)tetrasulfide, bis(2-monoethoxydimethylsilylethyl)trisulfide, bis(2-monoethoxydimethylsilylethyl)disulfide, and the like. Among these, bis(3-triethoxysilylpropyl)tetrasulfide is particularly preferable.

Examples of thioester silane coupling agents include 3-hexanoylthiopropyltriethoxysilane, 3-octanoylthiopropyltriethoxysilane, 3-decanoylthiopropyltriethoxysilane, 3-lauroylthiopropyltriethoxysilane, 2-hexanoylthioethyltriethoxysilane, 2-octanoylthioethyltriethoxysilane, 2-decanoylthioethyltriethoxysilane, 2-lauroylthioethyltriethoxysilane, 3-hexanoylthiopropyltrimethoxysilane, 3-octanoylthiopropyltrimethoxysilane, 3-decanoylthiopropyltrimethoxysilane, 3-lauroylthiopropyltrimethoxysilane, 2-hexanoylthioethyltrimethoxysilane, 2-octanoylthioethyltrimethoxysilane, 2-decanoylthioethyltrimethoxysilane, 2-lauroylthioethyltrimethoxysilane, and the like.

Examples of thiol silane coupling agents include 3-mercaptopropyltrimethoxysilane, 3-mercaptopropyltriethoxysilane, 3-mercaptopropylmethyldimethoxysilane, and the like.

Examples of olefin silane coupling agents include dimethoxymethylvinylsilane, vinyltrimethoxysilane, dimethylethoxyvinylsilane, diethoxymethylvinylsilane, triethoxyvinylsilane, vinyltris(2-methoxyethoxy)silane, allyltrimethoxysilane, allyltriethoxysilane, p-styryltrimethoxysilane, 3-(methoxydimethoxydimethylsilyl)propyl acrylate, 3-(trimethoxysilyl)propyl acrylate, 3-[dimethoxy(methyl)silyl]propyl methacrylate, 3-(trimethoxysilyl)propyl methacrylate, 3-[dimethoxy(methyl)silyl]propyl methacrylate, 3-(triethoxysilyl)propyl methacrylate, 3-[tris(trimethylsiloxy)silyl]propyl methacrylate, and the like.

Examples of epoxy silane coupling agents include 3-glycidyloxypropyl(dimethoxy)methylsilane, 3-glycidyloxypropyltrimethoxysilane, diethoxy(3-glycidyloxypropyl)methylsilane, triethoxy(3-glycidyloxypropyl)silane, 2-(3,4-epoxycyclohexyl)ethyltrimethoxysilane, and the like. Among these, 3-glycidyloxypropyltrimethoxysilane is preferable.

Examples of amino silane coupling agents include N-2-(aminoethyl)-3-aminopropylmethyldimethoxysilane, N-2-(aminoethyl)-3-aminopropyltrimethoxysilane, 3-aminopropyltrimethoxysilane, 3-aminopropyltriethoxysilane, 3-ethoxysilyl-N-(1,3-dimethylbutylidene)propylamine, N-phenyl-3-aminopropyltrimethoxysilane, N-(vinylbenzyl)-2-aminoethyl-3-aminopropyltrimethoxysilane, and the like. Among these, 3-aminopropyltriethoxysilane is preferable.

Examples of alkyl silane coupling agents include methyltrimethoxysilane, dimethyldimethoxysilane, trimethylmethoxysilane, methyltriethoxysilane, ethyltrimethoxysilane, n-propyltrimethoxysilane, isobutyltrimethoxysilane, isobutyltriethoxysilane, n-hexyltrimethoxysilane, n-hexyltriethoxysilane, cyclohexylmethyldimethoxysilane, n-octyltriethoxysilane, n-decyltrimethoxysilane, and the like. Among these, methyltriethoxysilane is preferable.

Among these silane coupling agents, bis(3-triethoxysilylpropyl)tetrasulfide can be particularly preferably used.

In the present invention, silane coupling agents can be used singly, or in a combination of two or more.

The amount of silane coupling agent in the rubber composition used in the present invention is preferably 0.1 to 20 parts by mass, and particularly preferably 3 to 15 parts by mass, per 100 parts by mass of the inorganic filler. This is because 0.1 parts by mass or more of a silane coupling agent can more advantageously improve low heat build-up of the rubber composition, whereas 20 parts by mass or less of a silane coupling agent can reduce the cost of the rubber composition and increase economic efficiency.

Use of rubber composition

The rubber composition of the present invention is used for at least one member selected from tread, sidewall, bead area, belt, carcass, and shoulder portions of a tire.

Method for producing the rubber composition

The method for producing the rubber composition used in the present invention is not particularly limited. The method for producing the rubber composition used in the present invention comprises the steps of: (A) kneading raw material ingredients including a rubber component, the additive of the present invention, and an inorganic filler and/or carbon black; and (B) kneading the mixture obtained in step (A) and a vulcanizing agent.

Step (A)

Step (A) is a step of kneading raw material ingredients including a rubber component, the additive of the present invention, and an inorganic filler and/or carbon black. It refers to the step before incorporating a vulcanizing agent.
In step (A), other ingredients as mentioned above etc. can also be incorporated, if necessary.
The kneading method in step (A) may be, for example, a method of kneading a composition comprising raw material ingredients including a rubber component, the additive of the present invention, and an inorganic filler and/or carbon black. In this kneading method, the entire amount of each ingredient may be kneaded at once, or each ingredient may be added in portions according to the intended purpose, such as viscosity adjustment, and kneaded. Alternatively, after kneading a rubber component and an inorganic filler and/or carbon black, the additive of the present invention may be added and kneaded; or, after kneading a rubber component and the additive of the present invention, an inorganic filler and/or carbon black may be added and kneaded. To uniformly disperse each ingredient, the kneading operation may be performed repeatedly.
Another example of the kneading method in step (A) is a two-step kneading method comprising the steps of (A-1) kneading a rubber component and the additive of the present invention; and (A-2) kneading the mixture (modified polymer) obtained in step (A-1) and raw material ingredients including an inorganic filler and/or carbon black (A-2).
The temperature of mixing the rubber composition in step (A) is not particularly limited. For example, the upper limit of the temperature of the rubber composition is preferably 120 to 190°C, more preferably 130 to 175°C, and still more preferably 140 to 170°C.
The mixing time in step (A) is not particularly limited. For example, the mixing time is preferably 10 seconds to 20 minutes, more preferably 30 seconds to 10 minutes, and more preferably 2 to 7 minutes.
The temperature of mixing the rubber component and the additive of the present invention in step (A-1) is preferably 80 to 190°C, more preferably 90 to 160°C, and still more preferably 100 to 150°C. This is because a mixing temperature of lower than 80°C does not allow the reaction to proceed, whereas a mixing temperature of 190°C or more accelerates deterioration of the rubber.
The mixing time in step (A-1) is preferably 10 seconds to 20 minutes, more preferably 30 seconds to 10 minutes, and still more preferably 60 seconds to 7 minutes. When the mixing time is shorter than 10 seconds, the reaction does not proceed sufficiently, whereas a mixing time of 20 minutes or more lowers the productivity.
The temperature of mixing the mixture (modified polymer) obtained in step (A-1) and an inorganic filler and/or carbon black in step (A-2) is not particularly limited. For example, the upper limit of the temperature of the mixture is preferably 120 to 190°C, more preferably 130 to 175°C, and still more preferably 140 to 170°C.
The mixing time in step (A-2) is not particularly limited. For example, the mixing time is preferably 10 seconds to 20 minutes, more preferably 30 seconds to 10 minutes, and still more preferably 2 to 7 minutes.
In step (A), the amount of the tetrazine compound (1) as the additive of the present invention is not particularly limited. For example, the amount of the tetrazine compound (1) is 0.1 to 10 parts by mass, preferably 0.25 to 5 parts by mass, and more preferably 0.5 to 2 parts by mass, per 100 parts by mass of the rubber component.
In step (A), the double bond portion of the rubber component (diene rubber) reacts with the additive of the present invention, i.e., a tetrazine compound (1), to form a modified polymer having six-membered structures represented by formulas (2) to (12), and thereby obtain a mixture in which an inorganic filler and/or carbon black is preferably dispersed.

Step (B)

Step (B) is a step of mixing the mixture obtained in step (A) and one or more vulcanizing agents. Step (B) means a final stage of kneading.
In step (B), a vulcanization accelerator etc. can also be added, if necessary.
Step (B) can be performed under heating conditions. The heating temperature in step (B) is not particularly limited. The temperature is preferably, for example, 60 to 140°C, more preferably 80 to 120°C, and still more preferably 90 to 120°C.
The mixing (or kneading) time is not particularly limited. For example, the mixing time is preferably 10 seconds to 20 minutes, more preferably 30 seconds to 10 minutes, and still more preferably 60 seconds to 5 minutes.
When the production process proceeds from step (A) to (B), it is preferable for the process to proceed to the

subsequent step (B) after the temperature is reduced by 30°C or more from the temperature after completion of the antecedent step.

In the method for producing the rubber composition used in the present invention, various ingredients usually incorporated in the rubber composition, for example, stearic acid, vulcanization accelerators such as zinc oxide, and antioxidants, may be added in step (A) or (B), if necessary.

The rubber composition used in the present invention may be mixed or kneaded using a Banbury mixer, a roll, an intensive mixer, a kneader, a twin-screw extruder, or the like. In an extrusion step, the resulting mixture is then extruded and processed to form, for example, a tread member or a sidewall member. Subsequently, the member is attached and molded in a usual manner using a tire molding machine to form a green tire. The green tire is heated under pressure in a vulcanizing machine to obtain a tire.

## 4. Tire

The tire of the present invention is produced using the above rubber composition comprising the modified polymer.

Examples of the tire of the present invention include pneumatic tires (such as radial-ply tires and bias tires), solid tires, and the like.

The use of the tire is not particularly limited. Examples include passenger car tires, heavy-duty tires, motorcycle tires, studless tires, and the like. Among these, the tire of the present invention is preferably used as passenger car tires.

The shape, structure, size, and material of the tire of the present invention are not particularly limited, and can be appropriately selected according to the purpose.

In the tire of the present invention, the above rubber composition comprising the modified polymer is used for at least one member selected from tread, sidewall, bead area, belt, carcass, and shoulder portions.

Among these, according to one preferable embodiment, a tire tread or sidewall part of a pneumatic tire is formed using the rubber composition.

The "tread" is a portion that has a tread pattern and comes into direct contact with the road surface. The tread is a tire casing portion for protecting the carcass, and preventing wear and flaws. The thread refers to a cap tread that constitutes the grounding part of a tire and/or to a base tread that is disposed inside the cap tread.

The "sidewall" refers to, for example, a portion from the lower side of a shoulder portion to a bead portion of a pneumatic radial-ply tire. Sidewall portions protect the carcass and are bent the most when the vehicle runs.

The "bead area" portions function to anchor both ends of carcass cords and simultaneously hold a tire to a rim. Beads are composed of bundles of high carbon steel.

The "belt" refers to a reinforcing band disposed between the carcass and the tread of a radial structure in the circumferential direction. The belt tightens the carcass like a hoop of a barrel to enhance the rigidity of the tread.

The "carcass" refers to a cord layer portion that forms the framework of a tire. The carcass plays a role in bearing the load, impact, and filled air pressure applied to the tire.

The "shoulder" refers to a shoulder portion of a tire. Shoulder portions play a role in protecting the carcass.

The tire of the present invention can be produced by methods known in the field of tires. The tire may be filled with ordinary air, or air having an adjusted oxygen partial pressure; or an inert gas, such as nitrogen, argon, or helium.

The tire of the present invention has low heat build-up and reduced rolling resistance, thus achieving lower fuel consumption of automobiles. Further, even the rubber composition highly filled with silica can have excellent low heat build-up, thus providing a fuel-efficient tire with high kinematic performance.

## Examples

The present invention is described below more specifically with reference to Production Examples and Examples. However, the following examples are only illustrative, and are not intended to limit the present invention thereto.

### Production Example 1: Production of 3,6-bis(3-pyridyl)-1,2,4,5-tetrazine (1a)

24 g (0.23 mol) of 3-cyanopyridine, 15 g (1.3 equivalents) of hydrazine hydrate, and 48 mL of methanol were placed in a 200-mL four-necked flask, and stirred at room temperature. Subsequently, 3.6 g (15 wt.%) of sulfur was added to this mixture. The flask was equipped with a condenser and the mixture was stirred overnight while heating at an outside temperature of 70°C. The reaction mixture was cooled with ice, and crystals were filtered and washed with a small amount of cold methanol. Crude crystals were dried under reduced pressure to obtain 19 g of orange dihydrotetrazine crude crystals.

17.8 g of the obtained crude crystals were dissolved in 178 g (40 equivalents) of acetic acid, and sulfur was removed by filtration. The resulting solution of dihydrotetrazine in acetic acid and 178 mL of distilled water were placed

in a 1-L four-necked recovery flask, and the mixture was stirred under ice-cooling. A solution of 15.5 g (3 equivalents) of sodium nitrite in 35 mL of distilled water was prepared and added dropwise to the reaction mixture over a period of about 1 hour. The resulting mixture was stirred overnight at room temperature. The precipitated crystals were filtered and neutralized with a 10% aqueous sodium bicarbonate solution to obtain crude crystals. The crude crystals were purified through a silica gel column (ethyl acetate) to obtain 8.4 g of the titled tetrazine compound (1a) (red-purple, acicular crystals).

Melting point: 200°C,
$^1$H-NMR (300 MHz, $CDCl_3$, $\delta$ ppm) :
7.59 (ddd, J = 0.9, 5.1, 7.8 Hz, 2 H), 8.89-8.96 (m, 4 H), 9.88 (dd, J = 0.9, 2.4 Hz, 2 H)

Production Example 2: Production of 3,6-diphenyl-1,2,4,5-tetrazine (1d)

120 g (1.16 mol) of benzonitrile, 76 g (1.3 equivalents) of hydrazine hydrate, and 348 mL of methanol were placed in a 500-mL four-necked flask and stirred at room temperature. Subsequently, 10 g (8.6 wt.%) of sulfur was added to this mixture. The flask was equipped with a condenser and the mixture was stirred overnight while heating at an outside temperature of 70°C. The obtained reaction mixture was cooled with ice, and the resulting crystals were filtered and washed with a small amount of cold methanol. The obtained crude crystals were dissolved in 2.5 L of warm methanol. After the insoluble matter was filtered, the solvent was distilled off from the filtrate. The obtained crude crystals were dried under reduced pressure to obtain 48 g of yellow dihydrotetrazine crude crystals.

4.8 g of the crude crystals, 48 mL of acetic acid, and 48 mL of distilled water were placed in a 300-mL four-necked recovery flask, and stirred under ice-cooling. 4.2 g (3 equivalents) of sodium nitrite was dissolved in 48 mL of distilled water. The solution was added dropwise to the reaction mixture over a period of about 1 hour, and the mixture was then stirred at room temperature overnight. 100 mL of distilled water was added to the reaction mixture, and crystals were filtered. The obtained crude crystals were washed with 10 mL of acetic acid and filtered to obtain 3.9 g of the titled diphenyl tetrazine compound (1d) (red-purple, acicular crystals).

Melting point: 166°C,
$^1$H-NMR (300 MHz, $CDCl_3$, $\delta$ ppm) :
7.58-7.68 (m, 6H), 8.64-8.69 (m, 4H)

Production Example 3: Production of 3,6-dibenzyl-1,2,4,5-tetrazine (1e)

58.5 g (0.5 mol) of phenylacetonitrile and 100 g (4.0 equivalents) of hydrazine hydrate were placed in a 300-mL four-necked flask and stirred at room temperature. Subsequently, 9.0 g (15 wt.%) of sulfur was added to this mixture. The flask was equipped with a condenser and the mixture was stirred overnight while heating at an outside temperature of 90°C. This reaction mixture was cooled with ice. After 100 mL of distilled water was added and the content was pulverized with a spatula, the crystals were filtered and washed with distilled water. The crude crystals were dried under reduced pressure to obtain 61 g of crude crystals containing white dihydrotetrazine.

61 g of the obtained crude crystals, 210 g of acetic acid, and 200 mL of distilled water were placed in a 1-L four-necked recovery flask, and the resulting mixture was stirred under ice-cooling. 23.9 g (1.5 equivalents) of sodium nitrite was dissolved in 100 mL of distilled water. The solution was added dropwise to the reaction mixture over a period of about 1 hour, and the resulting mixture was stirred at room temperature overnight. After 500 mL of distilled water was added to the reaction mixture, the resulting mixture was extracted with 100 mL of ethyl acetate three times. After the obtained organic layer was washed once with 100 mL of distilled water, once with 200 mL of a saturated aqueous sodium bicarbonate solution, and once with 200 mL of saturated saline, the solvent was distilled off to obtain 48 g of red crude crystals. The crude crystals were purified using a silica gel column (n-hexane:ethyl acetate = 5:1) to obtain 5.1 g of the titled tetrazine compound (1e) (red, flaky crystals).

Melting point: 68°C
$^1$H-1MR (300 MHz, $CDCl_3$, $\delta$ ppm) :
4.60 (s, 4H), 7.22-7.35 (m, 6H), 7.39-7.43 (m, 4H)

Production Example 4: Production of 3,6-bis(2-furanyl)-1,2,4,5-tetrazine (1f)

3 g (0.032 mol) of 2-furonitrile, 3.3 g (2.0 equivalents) of hydrazine hydrate, and 15 mL of ethanol were placed in a 50-mL three-necked flask. The resulting mixture was stirred under ice-cooling. Subsequently, 0.3 g (10 wt.%) of sulfur was added to this mixture. The flask was equipped with a condenser and the mixture was stirred for 2 hours while

heating at an outside temperature of 80°C. The obtained reaction mixture was cooled with ice. The crystals were filtered and then dried under reduced pressure to obtain 2.48 g of yellow dihydrotetrazine crude crystals.

2.48 g of the crude crystals, 150 mL of chloroform, and 35 mL of isoamyl nitrite were placed in a 500-mL four-necked recovery flask, and stirred at room temperature overnight. The solvent was removed by drying under reduced pressure and 2.39 g of the obtained crude crystals were purified through a silica gel column (chloroform:n-hexane = 3:1) to obtain 1.31 g of the titled tetrazine compound (1f) (red solid).

Melting point: 198 to 199°C,
$^1$H-NMR (500 MHz, $CDCl_3$, $\delta$ ppm) :
7.81 (dd, J = 0.4, 1.7 Hz, 2H), 7.67 (dd, J = 0.4, 3.6 Hz, 2H), 6.72 (dd, J = 1.7, 3.6 Hz, 2H)

Production Example 5: Production of 3-methyl-6-(3-pyridyl)-1,2,4,5-tetrazine (1g)

124.8 g (1.2 mol) of 3-cyanopyridine, 567.6 g (5.0 equivalents) of acetamidine hydrochloride, and 564 g (10.0 equivalents) of hydrazine hydrate were placed in a 2-L four-necked flask under ice-cooling. The resulting mixture was stirred at room temperature overnight. This reaction mixture was cooled with ice. The resulting crystals were filtered and dried under reduced pressure to obtain 431.2 g of crude crystals.

431.2 g of the crude crystals, 720 g (10 equivalents) of acetic acid, and 200 mL of distilled water were placed in a 5-L beaker, and stirred under ice-cooling. A solution of 300 g (3.7 equivalents) of sodium nitrite in 720 mL of distilled water was prepared and added dropwise to the reaction mixture over a period of about 1 hour. The mixture was stirred under ice-cooling for 1 hour. The reaction mixture was neutralized with an aqueous sodium bicarbonate solution, and extracted with ethyl acetate. The organic layer was then concentrated under reduced pressure to obtain 156.54 g of crude crystals. The crude crystals were purified using a silica gel column (n-hexane:ethyl acetate = 3:1) to obtain 71.81 g of the titled tetrazine compound (1g) (red-purple, crystals).

Melting point: 102°C,
$^1$H-NMR (500 MHz, $CDCl_3$, $\delta$ ppm) :
9.80 (m, J = 1.6 Hz, 1H), 8.84-8.87 (m, 2H), 7.55 (ddd and J = 0.7, 4.9, 8.0 Hz, 1H), 3.14 (s, 3H)

Production Example 6: Production of 3,6-bis(3,5-dimethyl-1-pyrazolyl)-1,2,4,5-tetrazine (lh)

250 g (2.26 mol) of aminoguanidine hydrochloride, 249 g (2.2 equivalents) of hydrazine hydrate, and 400 mL of methanol were placed in a 2000-mL four-necked flask, and heated under reflux for 24 hours. After the mixture was cooled to room temperature, the resulting solid was filtered and washed with methanol. The obtained solid was dried under reduced pressure to obtain 286 g of white triaminoguanidine hydrochloride (yield: 90%) .

150 g (1.07 mol) of the synthesized triaminoguanidine hydrochloride and 1250 mL of distilled water were placed in a 2000-mL four-necked flask. While the temperature in the flask was maintained at or below 30°C, 214 g (2.0 equivalents) of acetylacetone was added over a period of 20 minutes. The temperature in the flask was then raised to 70°C, and stirring was continued for 5 hours. After cooling to room temperature, the solid was filtered and washed with distilled water and n-hexane. The obtained solid was dried under reduced pressure to obtain 125 g (yield: 86%) of pale yellow dihydrotetrazine.

65 g (0.24 mol) of the synthesized dihydrotetrazine, 350 mL of distilled water, and 137 mL (10.0 equivalents) of acetic acid were placed in a 5000-mL beaker and cooled in an ice bath. A solution of 33 g (2.0 equivalents) of sodium nitrite in 50 mL of distilled water was prepared and added dropwise thereto. After stirring was continued in an ice bath for 2 hours, the temperature was raised to room temperature, and stirring was further continued for 4 hours. The resulting solid was then filtered, and washed with distilled water and n-hexane. The obtained solid was dried under reduced pressure to obtain 64 g (yield: 98%) of the titled tetrazine (1h) having red color.

Melting point: 220°C,
$^1$H-NMR (300 MHz, $CDCl_3$, $\delta$ ppm) :
2.40 (s, 6H), 2.72 (s, 6H), 6.20 (s, 2H)

Production Example 7: Production of 3,6-bis(2-thienyl)-1,2,4,5-tetrazine (1i)

21.48 g (0.197 mol) of 2-cyanothiophene, 4.3 g (20 wt.%) of sulfur, 92 mL of ethanol, and 20.1 g (2.1 equivalents) of hydrazine hydrate were placed in a 300-mL four-necked flask under ice-cooling and stirred at 65°C for 4 hours. This reaction mixture was cooled with ice. The resulting crystals were filtered, washed with distilled water, and then dried under reduced pressure to obtain 20.56 g of crude crystals.

20.56 g of the crude crystals, 59.1 g (5 equivalents) of acetic acid, and 60 mL of distilled water were placed in a 1-L beaker and stirred under ice-cooling. A solution of 40.7 g (3 equivalents) of sodium nitrite in 80 mL of distilled water was prepared and added dropwise to the reaction mixture over a period of about 1 hour. The resulting mixture was stirred under ice-cooling for 5 hours, and neutralized with an aqueous sodium bicarbonate solution. After the mixture was extracted with ethyl acetate, the organic layer was then concentrated under reduced pressure to obtain 18.7 g of crude crystals. The crude crystals were purified through a silica gel column (dichloromethane:n-hexane = 2:1) to obtain 16.8 g of the titled tetrazine compound (1i) (red, crystals).

Melting point: 198°C,
$^1$H-NMR (500 MHz, $CDCl_3$, $\delta$ ppm) :
8.28 (dd, J = 0.9, 3.8 Hz, 2H), 7.69 (dd, 0.9, 5.0 Hz, 2H), 7.28 (m, 2H)

Production Example 8: Production of 3-methyl,6-(2-pyridyl)-1,2,4,5-tetrazine (1j)

While a 100-mL four-necked flask was cooled with ice, 5 g (0.048 mol) of 2-cyanopyridine, 22.7 g (5.0 equivalents) of acetamidine hydrochloride, and 24 g (10.0 equivalents) of hydrazine hydrate were placed in the flask and stirred at room temperature overnight. The reaction mixture was cooled with ice and the obtained crystals were filtered. The crude crystals were dried under reduced pressure to obtain 14.15 g of crude crystals.

14.15 g of the crude crystals were placed in a 1-L beaker, and 42.5 g (15 equivalents) of acetic acid and 41 mL of distilled water were added thereto. The resulting mixture was stirred under ice-cooling. A solution of 32.2 g (10 equivalents) of sodium nitrite in 60 mL of distilled water was prepared and added dropwise to the reaction mixture over a period of about 1 hour. The resulting mixture was stirred under ice-cooling for 5 hours. The reaction mixture was neutralized with an aqueous sodium bicarbonate solution and extracted with ethyl acetate. The organic layer was then concentrated under reduced pressure to obtain 4.74 g of crude crystals. The crude crystals were purified through a silica gel column (n-hexane:ethyl acetate = 3:1) to obtain 1.02 g of the titled tetrazine compound (1j) (red, crystals).

Melting point: 63°C
$^1$H-NMR (500 MHz, $CDCl_3$, $\delta$ ppm) :
8.96 (m, 1H), 8.65 (m, 1H), 7.99 (ddd, J = 1.5, 7.8, 8.3 Hz, 1H), 7.57 (ddd, J = 0.7, 4.7, 7.8 Hz, 1H), 3.17 (s, 3H)

Production Example 9: Production of 3,6-bis(4-hydroxyphenyl)-1,2,4,5-tetrazine (1k)

50.0 g (0.42 mol) of 4-hydroxybenzonitrile and 63.0 g (3.0 equivalents) of hydrazine hydrate were placed in a 300-mL three-necked flask and stirred under ice-cooling. The resulting mixture was then heated with stirring at 70°C for 20 hours. The obtained reaction mixture was cooled with ice. The crystals were filtered and then dried under reduced pressure to obtain 49.8 g of yellow dihydrotetrazine crude crystals.

49.8 g of the crude crystals and 500 mL of chloroform were placed in a 1-L four-necked recovery flask. While the mixture was stirring at room temperature, oxygen was bubbled into the reaction mixture for 20 hours. After the mixture was filtered, the obtained crude crystals were recrystallized with DMF to obtain 52.0 g of the titled tetrazine compound (1k) (a red solid).

Melting point: 320°C (decomposition),
$^1$H-NMR (300 MHz, $CDCl_3$, $\delta$ ppm) :
8.36 (m, 4H), 7.03 (m, 4H)

Production Example 10: Production of 3,6-bis(3-hydroxyphenyl)-1,2,4,5-tetrazine (1l)

50 g (0.42 mol) of 3-cyanophenol, 42 g (2 equivalents) of hydrazine hydrate, and 5 g (10 wt.%) of sulfur were placed in a 300-mL four-necked flask and stirred at room temperature. The flask was then equipped with a condenser and the mixture was stirred overnight while heating at an outside temperature of 50°C. The obtained reaction mixture was cooled with ice. The resulting crystals were filtered and washed with a small amount of cold ethanol. The resulting crystals were dried under reduced pressure to obtain 21.5 g of dihydrotetrazine crude crystals.

21.5 g of the crude crystals and 430 mL of ethanol were placed in a 1-L recovery flask, and stirred at room temperature. While oxygen was bubbled into the reaction mixture, the mixture was stirred for 10 hours, and then concentrated under reduced pressure to obtain 21.5 g of crude crystals. The crude crystals were washed with ethanol and distilled water to obtain 7.3 g of the titled tetrazine compound (11) (orange, solid).

Melting point: 304 to 305.5°C,

$^1$H-NMR (500 MHz, $d_6$-DMSO, δ ppm) :
10.01 (s, 2H), 7.98 (dd, J = 1.6, 7.8 Hz, 2H), 7.94 (dd, J = 1.6, 1.8 Hz, 2H), 7.49 (dd, J = 7.8, 8.0 Hz, 2H), 7.09 (dd, J = 1.8, 8.0 Hz, 2H)

Production Example 11: Production of 3,6-bis(2-pyrimidinyl)-1,2,4,5-tetrazine (1m)

25 g (0.238 mol) of 2-cyanopyrimidine, 23.8 g (2 equivalents) of hydrazine hydrate, 28.6 g (2 equivalents) of acetic acid, and dimethyl sulfoxide (8.3 mL) were placed in a 200-mL four-necked flask. The resulting mixture was stirred at room temperature. The mixture was stirred overnight while heating at an outside temperature of 50°C. This reaction mixture was cooled with ice. The resulting crystals were filtered and dried under reduced pressure to obtain 30.1 g of dihydrotetrazine crude crystals.

30.1 g of the crude crystals, 500 mL of tetrahydrofuran, and 3.8 L (8 equivalents) of 0.5 N hydrochloric acid were placed in a 5-L beaker and stirred under ice-cooling. A solution of 32.8 g (2 equivalents) of sodium nitrite in 60 mL of distilled water was prepared and added dropwise to the reaction mixture over a period of about 0.5 hours. The mixture was stirred under ice-cooling for 1 hour, then extracted with methylene chloride, and concentrated under reduced pressure to obtain crude crystals. 3.4 g of the crude crystals were washed with 250 mL of acetone to obtain 3.2 g of the titled tetrazine compound (1m) (purple, solid).

Melting point: 264 to 267°C,
$^1$H-NMR (500 MHz, $CDCl_3$, δ ppm) :
9.18 (d, J = 4.9 Hz, 4H), 7.63 (t, J = 4.9 Hz, 2H)

Production Example 12: Production of 3,6-bis(2-pyrazinyl)-1,2,4,5-tetrazine (In)

25 g (0.238 mol) of cyanopyrazine, 23.8 g (2 equivalents) of hydrazine hydrate, 28.6 g (2 equivalents) of acetic acid, and 720 mL of methanol were placed in a 2-L four-necked flask and stirred at room temperature. The mixture was stirred overnight while heating at an outside temperature of 50°C. This reaction mixture was cooled with ice. The resulting crystals were filtered, washed with methanol, and then dried under reduced pressure to obtain 26.6 g of dihydrotetrazine crude crystals.

The half amount, i.e., 13.3 g, of the obtained dihydrotetrazine crude crystals, 400 mL of tetrahydrofuran, and 2.4 L (10 equivalents) of 0.5 N hydrochloric acid were placed in a 5-L beaker and stirred under ice-cooling. A solution of 24.6 g (3 equivalents) of sodium nitrite in 50 mL of distilled water was prepared and added dropwise to the reaction mixture over a period of about 0.5 hours. The mixture was stirred under ice-cooling for 1 hour, extracted with methylene chloride, and then concentrated under reduced pressure to obtain crude crystals. The same operation was performed using the remainder, i.e., the remaining half, of the crude crystals of the dihydrotetrazine compound. As a result, 19.1 g of tetrazine crude crystals were obtained. The crude crystals were dissolved in 960 mL of chloroform, and 320 mL of n-hexane was added to the solution. The mixture was filtered and the filtrate was concentrated under reduced pressure to obtain 11.9 g of the titled tetrazine compound (In) (red, solid).

Melting point: 208 to 210°C,
$^1$H-NMR (500 MHz, $CDCl_3$, δ ppm) :
9.97 (s, 2H), 8.98 (s, 2H), 8.92 (d, J = 2.1 Hz, 2H)

Production Examples 13 to 44: Production of modified polymer by kneading

The rubber component and the tetrazine compound in the proportions (parts by mass) shown in Tables 1 to 3 were kneaded using a Banbury mixer. When the temperature of the mixture had reached 130 to 150°C, the mixture was kneaded for about 2 minutes while maintain the temperature by adjustment. The resulting mixture was then cooled on a roll mill to produce a modified polymer.

Table 1

| | Production Example | | | | | | | | | | |
|---|---|---|---|---|---|---|---|---|---|---|---|
| | 13 | 14 | 15 | 16 | 17 | 18 | 19 | 20 | 21 | 22 | 23 |
| S-SBR*1 | 110 | 110 | 137.5 | 137.5 | 137.5 | | | 110 | 110 | 110 | 82.5 |
| Terminal-modified S-SBR*6 | | | | | | 100 | | | | | |

(continued)

| | Production Example | | | | | | | | | | |
|---|---|---|---|---|---|---|---|---|---|---|---|
| | 13 | 14 | 15 | 16 | 17 | 18 | 19 | 20 | 21 | 22 | 23 |
| BR*12 | | | | | | | 100 | 20 | 20 | 20 | 40 |
| Compound (1a)*51 | 1 | | | | | | | | | | |
| Compound (1b)*52 | | 1 | 2 | 125 | 3 | 2 | 2 | | | 1 | 1 |
| Compound (1j)*60 | | | | | | | | 1 | | | |
| Compound (1k)*61 | | | | | | | | | 1 | | |

Table 2

| | Production Example | | | | | | | | | |
|---|---|---|---|---|---|---|---|---|---|---|
| | 24 | 25 | 26 | 27 | 28 | 29 | 30 | 31 | 32 | 33 |
| S-SBR*1 | 110 | | | 110 | | | | | | |
| E-SBR*10 | | 137.5 | 137.5 | | | | | | | |
| E-SBR*11 | | | | 20 | | | | | | |
| BR*12 | 20 | | | | | | 40 | 40 | | |
| NR*13 | | | | | 100 | | 60 | | | |
| NR*14 | | | | | | 100 | | 60 | | |
| IR*17 | | | | | | | | | 100 | |
| NBR*18 | | | | | | | | | | 100 |
| Compound (1b)*52 | 2 | 1.25 | 2 | 1 | 1 | 1 | 0.6 | 0.6 | 1 | 1 |

Table 3

| | Production Example | | | | | | | | | | |
|---|---|---|---|---|---|---|---|---|---|---|---|
| | 34 | 35 | 36 | 37 | 38 | 39 | 40 | 41 | 42 | 43 | 44 |
| S-SBR*1 | 110 | 110 | 110 | 110 | 110 | | 110 | 110 | 110 | 82.5 | 82.5 |
| BR*12 | 20 | 20 | 20 | 20 | 20 | | 20 | 20 | 20 | 40 | 40 |
| CR*19 | | | | | | 100 | | | | | |
| Compound (1b)*52 | | | | 3 | 5 | 1 | 13 | 0.67 | 6.7 | 2 | 1.3 |
| Compound (1l*62 | 15 | | | | | | | | | | |
| Compound (1m)*63 | | 15 | | | | | | | | | |
| Compound (1n)*64 | | | 1.5 | | | | | | | | |

Description of symbols in tables

The raw materials used in the Examples (in the Tables) are as follows.

*1: solution-polymerized SBR (S-SBR), produced by PetroChina Dushanzi Petrochemical Company, trade name "RC2557S"
*2: solution-polymerized SBR (S-SBR), produced by Asahi Kasei Chemicals Corporation, trade name "Tafdene3835"
*3: solution-polymerized SBR (S-SBR), produced by LANXESS, trade name "Buna VSL 5025-2"
*4: solution-polymerized SBR (S-SBR), produced by LANXESS, trade name "Buna VSL 4526-2"

*5: solution-polymerized SBR (S-SBR), produced by LANXESS, trade name "Buna VSL 2538-2"
*6: terminal-modified solution-polymerized SBR (terminal-modified S-SBR), produced by Zeon Corporation, trade name "Nipol NS116R"
*7: terminal-modified solution-polymerized SBR (terminal-modified S-SBR), produced by Zeon Corporation, trade name "Nipol NS616"
*8: terminal-modified solution-polymerized SBR (terminal-modified S-SBR), produced by Asahi Kasei Chemicals Corporation, trade name "F3420"
*9: terminal-modified solution-polymerized SBR (terminal-modified S-SBR), produced by Asahi Kasei Chemicals Corporation, trade name "Asaprene Y031"
*10: emulsion-polymerized SBR (E-SBR), produced by Shenhua Chemical Industrial Co., Ltd., trade name "SBR1739"
*11: emulsion-polymerized SBR (E-SBR), produced by Zeon Corporation, trade name "Nipol 1502"
*12: butadiene rubber (BR), produced by Sinopec Qilu Petrochemical Co., Ltd., trade name "BR9000"
*13: natural rubber (NR), produced by Guangken Rubber Co., Ltd., trade name "TSR20"
*14: natural rubber (NR), produced by Sinochem International Corp., trade name "RSS3"
*15: isoprene rubber (IR), produced by Sterlitamak Kauchuk CSC, trade name "IR-1"
*16: isoprene rubber (IR), produced by Sterlitamak, trade name "IR-2"
*17: isoprene rubber (IR), produced by Sterlitamak, trade name "SKI-3"
*18: nitrile rubber (NBR), produced by Zeon Corporation, trade name "NBR3350"
*19: chloroprene rubber (CR), produced by Mitsui Plastics Trading Co. Ltd., trade name "DCR40A"
*20: carbon black, produced by Cabot, trade name "N234"
*21: carbon black, produced by Cabot, trade name "N330"
*22: carbon black, produced by Cabot, trade name "N375"
*23: carbon black, produced by Cabot, trade name "N550"
*24: produced by Quechen Silicon Chemical Co. Ltd., trade name "HD60MP"
*25: produced by Quechen Silicon Chemical Co., Ltd., trade name "HD90MP"
*26: produced by Quechen Silicon Chemical Co., Ltd., trade name "HD115MP"
*27: produced by Quechen Silicon Chemical Co., Ltd., trade name "HD165MP"
*28: produced by Quechen Silicon Chemical Co., Ltd., trade name "HD200MP"
*29: produced by Quechen Silicon Chemical Co., Ltd., trade name "HD250MP"
*30: produced by Evonik Industries AG, trade name "Si69"
*31: produced by Zhangjiagang Guotai-Huarong New Chemical Materials Co., Ltd., trade name "SCA-1113"
*32: produced by Zhangjiagang Guotai-Huarong New Chemical Materials Co., Ltd., trade name "SCA-113"
*33: produced by Zhangjiagang Guotai-Huarong New Chemical Materials Co., Ltd., trade name "SCA-403"
*34: produced by Kemai Chemical Co., Ltd., trade name "6-PPD"
*35: produced by Kemai Chemical Co., Ltd., trade name "DPG"
*36: produced by Kemai Chemical Co., Ltd., trade name "CBS"
*37: produced by Kemai Chemical Co., Ltd., trade name "TMQ"
*38: produced by Kemai Chemical Co., Ltd., trade name "DM"
*39: produced by AkzoNobel, trade name "DCP"
*40: produced by Kemai Chemical Co., Ltd., trade name "TMTD"
*41: produced by Rhein Chemie Rheinau GmbH, trade name "Antilux 111"
*42: stearic acid, produced by Sichuan Tianyu Grease Chemical Co., Ltd.
*43: zinc oxide, produced by Dalian Zinc Oxide Co., Ltd.
*44: magnesium oxide, produced by Xingtai Meishen Industries Co., Ltd.
*45: sulfur, produced by Shanghai Jinghai Chemical Co. Ltd.
*46: produced by Hansen & Rosenthal, trade name "Vivatec 500"
*47: produced by Hansen & Rosenthal, trade name "Vivatec 700"
*48: produced by Jiangsu Hongxin Chemical Co., Ltd., trade name "DOP"
*49: produced by Zhejiang Huangyan Zhedong Rubber Chemicals Co., Ltd., trade name "MB"
*50: produced by Chemtura Corp., Ltd., trade name "OCTAMINE"
*51: tetrazine compound (1a): 3,6-bis(3-pyridyl)-1,2,4,5-tetrazine (compound produced in Production Example 1)
*52: tetrazine compound (1b): 3,6-bis(2-pyridyl)-1,2,4,5-tetrazine, produced by Tokyo Chemical Industry, Co., Ltd.
*53: tetrazine compound (1c): 3,6-bis(4-pyridyl)-1,2,4,5-tetrazine, produced by Tokyo Chemical Industry, Co., Ltd.
*54: tetrazine compound (1d): 3,6-diphenyl-1,2,4,5-tetrazine (compound produced in Production Example 2)
*55: tetrazine compound (1e): 3,6-dibenzyl-1,2,4,5-tetrazine (compound produced in Production Example 3)
*56: tetrazine compound (1f): 3,6-bis(2-furanyl)-1,2,4,5-tetrazine (compound produced in Production Example 4)
*57: tetrazine compound (1g): 3-methyl-6-(3-pyridyl)-1,2,4,5-tetrazine (compound produced in Production Example

5)
*58: tetrazine compound (1h): 3,6-bis(3,5-dimethyl-l-pyrazolyl)-1,2,4,5-tetrazine (compound produced in Production Example 6)
*59: tetrazine compound (1i): 3,6-bis(2-thienyl)-1,2,4,5-tetrazine (compound produced in Production Example 7)
*60: tetrazine compound (1j): 3-methyl-6-(2-pyridyl)-1,2,4,5-tetrazine (compound produced in Production Example 8)
*61: tetrazine compound (1k): 3,6-bis(4-hydroxyphenyl)-1,2,4,5-tetrazine (compound produced in Production Example 9)
*62: tetrazine compound (1l): 3,6-bis(3-hydroxyphenyl)-1,2,4,5-tetrazine (compound produced in Production Example 10)
*63: tetrazine compound (1m): 3,6-bis(2-pyrimidinyl)-1,2,4,5-tetrazine (compound produced in Production Example 11)
*64: tetrazine compound (1n): 3,6-bis(2-pyrazinyl)-1,2,4,5-tetrazine (compound produced in Production Example 12)
*65: modified S-SBR produced in Production Example 13
*66: modified S-SBR produced in Production Example 14
*67: modified S-SBR produced in Production Example 15
*68: modified S-SBR produced in Production Example 16
*69: modified S-SBR produced in Production Example 17
*70: modified S-SBR produced in Production Example 18
*71: modified BR produced in Production Example 19
*72: modified S-SBR·BR produced in Production Example 20
*73: modified S-SBR·BR produced in Production Example 21
*74: modified S-SBR·BR produced in Production Example 22
*75: modified S-SBR·BR produced in Production Example 23
*76: modified S-SBR·BR produced in Production Example 24
*77: modified E-SBR produced in Production Example 25
*78: modified E-SBR produced in Production Example 26
*79: modified S-SBR·E-SBR produced in Production Example 27
*80: modified NR produced in Production Example 28
*81: modified NR produced in Production Example 29
*82: modified NR·BR produced in Production Example 30
*83: modified NR·BR produced in Production Example 31
*84: modified IR produced in Production Example 32
*85: modified NBR produced in Production Example 33
*86: modified S-SBR·BR produced in Production Example 34
*87: modified S-SBR·BR produced in Production Example 35
*88: modified S-SBR·BR produced in Production Example 36
*89: modified S-SBR·BR produced in Production Example 37
*90: modified S-SBR·BR produced in Production Example 38
*91: modified CR produced in Production Example 39
*92: modified S-SBR·BR produced in Production Example 40
*93: modified S-SBR·BR produced in Production Example 41
*94: modified S-SBR·BR produced in Production Example 42
*95: modified S-SBR·BR produced in Production Example 43
*96: modified S-SBR·BR produced in Production Example 44

Production Example 45: Production of tetrazine-modified polymer and confirmation of its structure

(1) S-SBR*2 (100 parts by mass) and the tetrazine compound (1b) (5 parts by mass) were kneaded using a Banbury mixer. After the temperature of the mixture had reached 130 to 150°C, kneading was continued for about 2 minutes, while maintaining the temperature by adjustment. The mixture was then cooled on a roll mill to produce a modified polymer (modified S-SBR).

(2) Tetrazine compound (1b), S-SBR, and modified S-SBR extracted with THF were dissolved in $CDCl_3$ to measure $^{13}C$-NMR. Fig. 1 shows measurement results of the tetrazine compound (1b). Figs. 2 and 3 show the measurement results of S-SBR. Figs. 4 and 5 show the measurement results of modified S-SBR extracted with THF. Further, Fig. 6 shows a comparison of $^{13}C$-NMR spectrum charts of the tetrazine compound (1b), S-SBR, and modified S-SBR.

Fig. 6 shows that the peak of the tetrazine compound (1b) disappears and new peaks suggesting the presence of

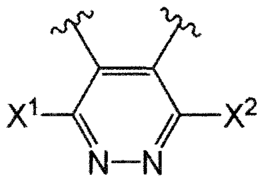 (2) 、 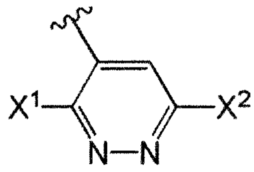 (3) 、 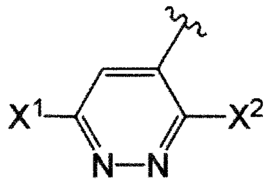 (4)

were confirmed. The above results clearly show that an inverse electron-demand Aza-Diels-Alder reaction proceeds between the tetrazine compound (1b) and a double bond of SBR.

Tetrazine compounds are red to purple compounds. The color specific to tetrazine disappears when the tetrazine compounds are kneaded with SBR. The color specific to tetrazine also disappears even when polymers other than SBR shown in the Production Examples of tetrazine modified polymers are used. The results thus show that the inverse electron-demand Aza-Diels-Alder reaction between the tetrazine compound and double bonds of the polymer proceeds.

Examples 1 to 129

The components shown in step (A) of Tables 4 to 13 below were mixed in the proportions (parts by weight) shown in the tables and kneaded using a Banbury mixer for 5 minutes, while adjusting the number of rotations so that the maximum temperature of the mixture was 160°C. After the obtained mixture was allowed to rest at 80°C or less, the components shown in step (B) of Tables 4 to 11 were added in the proportions (parts by weight) shown in the tables to the mixer and kneaded while controlling the temperature so that the maximum temperature of the mixture did not exceed 110°C. Each rubber composition was thus obtained.

Examples 130 to 133

The components shown in step (A-1) of Table 14 below were mixed in the proportions (parts by mass) shown in the table and kneaded using a Banbury mixer for the time shown in Table 14 (kneading time), while adjusting the number of rotations to maintain the temperature (mixture temperature) shown in Table 14. The components shown in step (A-2) of Table 14 were placed in the proportions shown therein and kneaded for 4 minutes while adjusting the temperature of the mixture to 160°C. After the mixture was allowed to rest until the temperature of the mixture became 80°C or less, the components shown in step (B) of Table 14 were added in the proportions shown in the table and kneaded using a Banbury mixer for 1 minute while adjusting the number of rotations such that the maximum temperature did not exceed 110°C. Each rubber composition was thus produced.

Table 4

| Components (parts by mass) | | | Example | | | | | | | | | | | | | | | | | | | |
|---|---|---|---|---|---|---|---|---|---|---|---|---|---|---|---|---|---|---|---|---|---|---|
| | | | 1 | 2 | 3 | 4 | 5 | 6 | 7 | 8 | 9 | 10 | 11 | 12 | 13 | 14 | 15 | 16 | 17 | 18 | 19 | 20 |
| Components of the rubber composition | Step (A) | S-SBR*1 | 137.5 | 137.5 | 137.5 | 137.5 | 137.5 | 137.5 | 137.5 | 137.5 | 137.5 | 110 | 110 | 110 | 110 | 110 | 110 | 110 | 110 | 110 | 110 | 110 |
| | | BR*12 | | | | | | | | | | 20 | 20 | 20 | 20 | 20 | 20 | 20 | 20 | 20 | 20 | 20 |
| | | Carbon black*20 | 4 | 4 | 6.4 | 4 | 4 | 4 | 4 | 4 | 4 | 4 | 4 | 4 | 4 | 4 | 4 | 4 | 4 | 4 | 4 | 4 |
| | | Silica*27 | 80 | 80 | 80 | 80 | 80 | 80 | 80 | 80 | 80 | 80 | 80 | 80 | 80 | 80 | 80 | 80 | 80 | 80 | 80 | 80 |
| | | Silane coupling agent*30 | 6.4 | 6.4 | 6.4 | 3.2 | | 6.4 | 6.4 | 6.4 | 6.4 | 6.4 | 6.4 | 6.4 | 6.4 | 6.4 | 6.4 | 6.4 | 6.4 | 6.4 | 6.4 | 6.4 |
| | | Compound (1a)*51 | 0.5 | 1 | 2 | 1 | 2 | | | | | 0.5 | 1 | 1 | | | | | | | | |
| | | Compound (1b)*52 | | | | | | 0.5 | 1 | | | | | | 1 | 1 | | | | | | |
| | | Compound (1c)*53 | | | | | | | | 1 | | | | | | | 1 | | | | | |
| | | Compound (1d)*54 | | | | | | | | | 1 | | | | | | | | | | | |
| | | Compound (1e)*55 | | | | | | | | | | | | | | | | 1 | | | | |
| | | Compound (1f)*56 | | | | | | | | | | | | | | | | | 1 | | | |
| | | Compound (1g)*57 | | | | | | | | | | | | | | | | | | 0.5 | 1 | |
| | | Compound (1h)*58 | | | | | | | | | | | | | | | | | | | | 1 |
| | | Wax*41 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 |
| | | Oil*47 | | | | | | | | | | 7.5 | 7.5 | 7.5 | 7.5 | 7.5 | 7.5 | 7.5 | 7.5 | 7.5 | 7.5 | 7.5 |
| | | Stearic acid*42 | 2 | 2 | 2 | 2 | 2 | 2 | 2 | 2 | 2 | 2 | 2 | 2 | 2 | 2 | 2 | 2 | 2 | 2 | 2 | 2 |
| | | Zinc oxide*43 | 2 | 2 | 2 | 2 | 2 | 2 | 2 | 2 | 2 | 2 | 2 | | 2 | | 2 | 2 | 2 | 2 | 2 | 2 |
| | | Antioxidant*34 | 2 | 2 | 2 | 2 | 2 | 2 | 2 | 2 | 2 | 2 | 2 | 2 | 2 | 2 | 2 | 2 | 2 | 2 | 2 | 2 |
| | Step (B) | Zinc oxide*43 | | | | | | | | | | | | 2 | | 2 | | | | | | |
| | | Vulcanization accelerator*35 | 1 | 1 | 1 | 1 | 1 | 1 | 1 | 1 | 1 | 1 | 1 | 1 | 1 | 1 | 1 | 1 | 1 | 1 | 1 | 1 |
| | | Vulcanization accelerator*36 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 |
| | | Sulfur*45 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 |

Table 5

| Components (parts by mass) | | | Example | | | | | | | | | | | | |
|---|---|---|---|---|---|---|---|---|---|---|---|---|---|---|---|
| | | | 21 | 22 | 23 | 24 | 25 | 26 | 27 | 28 | 29 | 30 | 31 | 32 | 33 |
| Components of the rubber composition | Step (A) | S-SBR*1 | 96.25 | 82.5 | | | | | | | 110 | 110 | 110 | 110 | |
| | | S-SBR*2 | | | | | | | | | | | | | 110 |
| | | S-SBR*3 | | | 110 | | | 82.5 | 82.5 | 82.5 | | | | | |
| | | S-SBR*4 | | | | 110 | | | | | | | | | |
| | | S-SBR*5 | | | | | 110 | | | | | | | | |
| | | BR*12 | 30 | 40 | 20 | 20 | 20 | 40 | 40 | 40 | 20 | 20 | 20 | 20 | 20 |
| | | Carbon black*20 | 4 | 4 | 4 | 4 | 4 | 4 | 4 | 4 | 5.6 | 5.6 | 5.6 | 5.6 | 5.6 |
| | | Silica*27 | 80 | 80 | 80 | 80 | 80 | 80 | 65 | 50 | 70 | 70 | 70 | 70 | 70 |
| | | Silane coupling agent*30 | 6.4 | 6.4 | 6.4 | 6.4 | 6.4 | 6.4 | 5.2 | 4 | 5.6 | 5.6 | 5.6 | 5.6 | 5.6 |
| | | Compound (1a)*51 | 1 | 1 | 1 | 1 | 1 | 1 | 1 | 1 | 1 | | | | 1 |
| | | Compound (1b)*52 | | | | | | | | | | 1 | | | |
| | | Compound (1c)*53 | | | | | | | | | | | 1 | | |
| | | Compound (1d)*54 | | | | | | | | | | | | 1 | |
| | | Wax*41 | 1.5 | 1.5 | 1.5 | 1.5 | 1.5 | 1.5 | 1.5 | 1.5 | 1.5 | 1.5 | 1.5 | 1.5 | 1.5 |
| | | Oil*47 | 11.25 | 15 | | | | | | | | | | | |
| | | Oil*46 | | | 7.5 | 7.5 | 7.5 | 15 | | | | | | | |
| | | Stearic acid*42 | 2 | 2 | 2 | 2 | 2 | 2 | 2 | 2 | 2 | 2 | 2 | 2 | 2 |
| | | Zinc oxide*43 | 2 | 2 | 2 | 2 | 2 | 2 | 2 | 2 | 2 | 2 | 2 | 2 | 2 |
| | | Antioxidant*34 | 2 | 2 | 2 | 2 | 2 | 2 | 2 | 2 | 2 | 2 | 2 | 2 | 2 |
| | Step (B) | Vulcanization accelerator*35 | 1 | 1 | 1 | 1 | 1 | 1 | 1 | 1 | 1 | 1 | 1 | 1 | 1 |
| | | Vulcanization accelerator*36 | 1.5 | 1.5 | 1.5 | 1.5 | 1.5 | 1.5 | 1.5 | 1.5 | 1.5 | 1.5 | 1.5 | 1.5 | 1.5 |
| | | Sulfur*45 | 1.5 | 1.5 | 1.5 | 1.5 | 1.5 | 1.5 | 1.5 | 1.5 | 1.5 | 1.5 | 1.5 | 1.5 | 1.5 |

Table 6

| Components (parts by mass) | | | Example 34 | 35 | 36 | 37 | 38 | 39 | 40 | 41 | 42 |
|---|---|---|---|---|---|---|---|---|---|---|---|
| Components of the rubber composition | Step (A) | S-SBR*1 | 110 | 110 | 110 | 110 | 110 | | | | |
| | | Terminal-modified S-SBR*7 | | | | | | 80 | 80 | | |
| | | Terminal-modified S-SBR*8 | | | | | | | | 100 | |
| | | Terminal-modified S-SBR*9 | | | | | | | | | 80 |
| | | BR*12 | 20 | 20 | 20 | 20 | 20 | 20 | 20 | 20 | 20 |
| | | Carbon black*20 | 4 | 4 | 4 | 4 | 4 | | 4 | 5.6 | 5.6 |
| | | Carbon black*21 | | | | | | 4 | | | |
| | | Silica*27 | | | 70 | | | | 50 | 70 | 70 |
| | | Silica*25 | 70 | | | | | | | | |
| | | Silica*26 | | 70 | | | | 65 | | | |
| | | Silica*28 | | | | 70 | | | | | |
| | | Silica*29 | | | | | 70 | | | | |
| | | Silane coupling agent*30 | 3.15 | 4.2 | 5.6 | 7 | 8.75 | 4 | 4 | 5.6 | 5.6 |
| | | Compound (1a)*51 | 1 | 1 | 1 | 1 | 1 | 0.5 | 1 | 1 | 1 |
| | | Wax*41 | 1.5 | 1.5 | 1.5 | 1.5 | 1.5 | 1.5 | 1.5 | 1.5 | 1.5 |
| | | Oil*47 | | | | | | 12 | | 10 | |
| | | Oil*46 | | | | | | | | | 30 |
| | | Stearic acid*42 | 2 | 2 | 2 | 2 | 2 | 2 | 2 | 2 | 2 |
| | | Zinc oxide*43 | 2 | 2 | 2 | 2 | 2 | 2 | 2 | 2 | 2 |
| | | Antioxidant*34 | 2 | 2 | 2 | 2 | 2 | 2 | 2 | 2 | 2 |
| | Step (B) | Vulcanization accelerator*35 | 1 | 1 | 1 | 1 | 1 | 1 | 1 | 1 | 1 |
| | | Vulcanization accelerator*36 | 1.5 | 1.5 | 1.5 | 1.5 | 1.5 | 1.5 | 1.5 | 1.5 | 1.5 |
| | | Sulfur*45 | 1.5 | 1.5 | 1.5 | 1.5 | 1.5 | 1.5 | 1.5 | 1.5 | 1.5 |

Table 7

| | | Components (parts by mass) | Example | | | | | | | | | | | | | | | | | | |
|---|---|---|---|---|---|---|---|---|---|---|---|---|---|---|---|---|---|---|---|---|---|
| | | | 43 | 44 | 45 | 46 | 47 | 48 | 49 | 50 | 51 | 52 | 53 | 54 | 55 | 56 | 57 | 58 | 59 | 60 | 61 |
| Components of the rubber composition | Step (A) | S-SBR*1 | | | | | | | | | | 110 | 110 | 110 | 137.5 | 110 | 110 | 110 | 110 | 110 | |
| | | E-SBR*10 | 137.5 | 137.5 | 137.5 | 137.5 | 110 | | | | | | | | | | | | | | |
| | | NR*13 | | | | | | 100 | 50 | 60 | | | | | | | | | | | |
| | | BR*12 | | | | | 20 | | 50 | 40 | | 20 | 20 | 20 | | 20 | 20 | 20 | 20 | 20 | |
| | | IR*15 | | | | | | | | | | | | | | | | | | | 100 |
| | | IR*16 | | | | | | | | | 100 | | | | | | | | | | |
| | | Carbon black*20 | 4 | 4 | 4 | 4 | 5.6 | 4 | 4 | 4 | | 24 | 44 | 64 | 80 | 84 | 84 | 84 | 84 | 84 | 55 |
| | | Silica*27 | 80 | 80 | 80 | 80 | 70 | 80 | 50 | 80 | 55 | 60 | 40 | 20 | | | | | | | |
| | | Silane coupling agent*30 | 6.4 | 6.4 | 6.4 | 6.4 | 5.6 | 6.4 | 4 | 6.4 | 4.4 | 4.8 | 3.2 | 1.6 | | | | | | | |
| | | Compound (1a)*51 | 0.5 | 1 | | | 1 | | 1 | | 1 | 1 | 1 | 1 | 2 | 1 | | | | | 1 |
| | | Compound (1b)*52 | | | 0.5 | 1 | | 1 | | 1 | | | | | | | | | | | |
| | | Compound (1c)*53 | | | | | | | | | | | | | | | 1 | | | | |
| | | Compound (1d)*54 | | | | | | | | | | | | | | | | 1 | | | |
| | | Compound (1f)*56 | | | | | | | | | | | | | | | | | 1 | | |
| | | Compound (1i)*59 | | | | | | | | | | | | | | | | | | 1 | |
| | | Wax*41 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 |
| | | Oil*47 | | | | | | 30 | | 30 | | 7.5 | 7.5 | 7.5 | | 7.5 | 7.5 | 7.5 | 7.5 | 7.5 | |
| | | Stearic acid*42 | 2 | 2 | 2 | 2 | 2 | 2 | 2 | 2 | 2 | 2 | 2 | 2 | 2 | 2 | 2 | 2 | 2 | 2 | 2 |
| | | Zinc oxide*43 | 2 | 2 | 2 | 2 | 2 | | 2 | | 2 | 2 | 2 | 2 | 2 | 2 | | | | | 2 |
| | | Antioxidant*34 | 2 | 2 | 2 | 2 | 2 | 2 | 2 | 2 | 2 | 2 | 2 | 2 | 2 | 2 | 2 | 2 | 2 | 2 | 2 |
| | Step (B) | Zinc oxide*43 | | | | | | 2 | | 2 | | | | | | | 2 | 2 | 2 | 2 | |
| | | Vulcanization accelerator*35 | 1 | 1 | 1 | 1 | 1 | 1 | 1 | 1 | 1 | 1 | 0.8 | 0.6 | | | | | | | |
| | | Vulcanization accelerator*36 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 |
| | | Sulfur*45 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 |

Table 8

| Components (parts by mass) | | | Example | | | | | | | | | | | | | | | | |
|---|---|---|---|---|---|---|---|---|---|---|---|---|---|---|---|---|---|---|---|
| | | | 62 | 63 | 64 | 65 | 66 | 67 | 68 | 69 | 70 | 71 | 72 | 73 | 74 | 75 | 76 | 77 | 78 |
| Components of the rubber composition | Step (A) | Modified S-SBR*65 | 111 | | 55.5 | | | | | | | | | | | | | | |
| | | Modified S-SBR*66 | | 111 | | 55.5 | | | | | | | | | | | | | |
| | | Modified S-SBR*67 | | | | | 110 | 80 | 60 | 40 | 60 | 60 | | | | | | | |
| | | Modified S-SBR*68 | | | | | | | | | | | 80 | 60 | | | | | |
| | | Modified S-SBR*69 | | | | | | | | | | | | | 80 | 60 | | | |
| | | Modified S-SBR*70 | | | | | | | | | | | | | | | 50 | 35 | |
| | | Modified BR*71 | | | | | | | | | | | | | | | | | 20 |
| | | BR*12 | 20 | 20 | 20 | 20 | 20 | 20 | 20 | 20 | 20 | 20 | 20 | 20 | 20 | 20 | 20 | 20 | |
| | | S-SBR*1 | | | 55 | 55 | | 30 | 50 | 70 | 50 | 50 | 30 | 50 | 30 | 50 | | | 110 |
| | | Terminal-modified S-SBR*6 | | | | | | | | | | | | | | | 30 | 45 | |
| | | Carbon black*20 | 4 | 4 | 4 | 4 | 4 | 4 | 4 | 4 | 4 | 4 | 4 | 4 | 4 | 4 | 4 | 4 | 4 |
| | | Silica*27 | 80 | 80 | 80 | 80 | 80 | 80 | 80 | 80 | 80 | 80 | 80 | 80 | 80 | 80 | 80 | 80 | 80 |
| | | Silane coupling agent*30 | 6.4 | 6.4 | 6.4 | 6.4 | 6.4 | 6.4 | 6.4 | 6.4 | 6.4 | 6.4 | 6.4 | 6.4 | 6.4 | 6.4 | 6.4 | 6.4 | 6.4 |
| | | Wax*41 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 |
| | | Oil*47 | 7.5 | 7.5 | 7.5 | 7.5 | 7.5 | 7.5 | 7.5 | 7.5 | 7.5 | 7.5 | 7.5 | 7.5 | 7.5 | 7.5 | 37.5 | 37.5 | 7.5 |
| | | Stearic acid*42 | 2 | 2 | 2 | 2 | 2 | 2 | 2 | 2 | 2 | 2 | 2 | 2 | 2 | 2 | 2 | 2 | 2 |
| | | Zinc oxide*43 | 2 | 2 | 2 | 2 | 2 | 2 | 2 | 2 | 2 | | 2 | 2 | 2 | 2 | 2 | 2 | 2 |
| | | Antioxidant*34 | 2 | 2 | 2 | 2 | 2 | 2 | 2 | 2 | 2 | 2 | 2 | 2 | 2 | 2 | 2 | 2 | 2 |
| | Step (B) | Zinc oxide*43 | | | | | | | | | | 2 | | | | | | | |
| | | Vulcanization accelerator*35 | 1 | 1 | 1 | 1 | 1 | 1 | 1 | 1 | 1 | 1 | 1 | 1 | 1 | 1 | 1 | 1 | 1 |
| | | Vulcanization accelerator*36 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 |
| | | Sulfur*45 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 |

Table 9

| Components (parts by mass) | | | Example | | | | | | | | | | | | | | |
|---|---|---|---|---|---|---|---|---|---|---|---|---|---|---|---|---|---|
| | | | 79 | 80 | 81 | 82 | 83 | 84 | 85 | 86 | 87 | 88 | 89 | 90 | 91 | 92 | 93 |
| Components of the rubber composition | Step (A) | Modified S-SBR/BR*72 | 131 | | | | | | | | | | | | | | |
| | | Modified S-SBR/BR*73 | | 131 | | | | | | | | | | | | | |
| | | Modified S-SBR/BR*74 | | | 131 | | | | 131 | | | | | | | | |
| | | Modified S-SBR/BR*75 | | | | 123.5 | 123.5 | 123.5 | | | | | | | | | |
| | | Modified S-SBR/BR*76 | | | | | | | | 65 | | | | | | | |
| | | Modified E-SBR*77 | | | | | | | | | 110 | 110 | 110 | | | | 96.25 |
| | | Modified E-SBR*78 | | | | | | | | | | | | 80 | 60 | | |
| | | Modified S-SBR・E-SBR*79 | | | | | | | | | | | | | | 91 | |
| | | BR*12 | | | | | | | | 10 | 20 | 20 | 20 | 20 | 20 | 20 | 20 |
| | | S-SBR*1 | | | | | | | | 55 | | | | | | | |
| | | E-SBR*10 | | | | | | | | | | | | 30 | 50 | | |
| | | NR*13 | | | | | | | | | | | | | | 10 | 10 |
| | | Carbon black*20 | 4 | 4 | 4 | 4 | 4 | 4 | 4 | 4 | 4 | | 24 | 4 | 4 | 4 | 24 |
| | | Carbon black*22 | | | | | | | | | | 24 | | | | | |
| | | Silica*27 | 80 | 80 | 80 | 80 | 65 | 50 | 80 | 80 | 70 | 50 | 50 | 80 | 80 | 60 | 50 |
| | | Silane coupling agent*30 | 6.4 | 6.4 | 6.4 | 6.4 | 5.2 | 4 | 6.4 | 6.4 | 5.6 | 4 | 4 | 6.4 | 6.4 | 4.8 | 4 |
| | | Wax*41 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 |
| | | Oil*47 | 7.5 | 7.5 | 7.5 | 15 | | | 7.5 | 7.5 | | | | 7.5 | 7.5 | | |
| | | Stearic acid*42 | 2 | 2 | 2 | 2 | 2 | 2 | 2 | 2 | 2 | 2 | 2 | 2 | 2 | 2 | 2 |
| | | Zinc oxide*43 | | | | 2 | 2 | 2 | 2 | 2 | 2 | 2 | 2 | 2 | 2 | | 2 |
| | | Antioxidant*34 | 2 | 2 | 2 | 2 | 2 | 2 | 2 | 2 | 2 | 2 | 2 | 2 | 2 | 2 | 2 |
| | Step (B) | Zinc oxide*43 | 2 | 2 | 2 | | | | | | | | | | | 2 | |
| | | Vulcanization accelerator*35 | 1 | 1 | 1 | 1 | 1 | 1 | 1 | 1 | 1 | 1 | 1 | 1 | 1 | 1 | 1 |
| | | Vulcanization accelerator*36 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 |
| | | Sulfur*45 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 |

Table 10

| Components (parts by mass) | | | Example | | | | | | | | | | | | |
|---|---|---|---|---|---|---|---|---|---|---|---|---|---|---|---|
| | | | 94 | 95 | 96 | 97 | 98 | 99 | 100 | 101 | 102 | 103 | 104 | 105 | 106 |
| Components of the rubber composition | Step (A) | Modified BR*71 | | | | | | 50 | | | | 50 | | | |
| | | Modified S-SBR/BR*74 | | | | | | | | | 131 | | | | |
| | | Modified NR*80 | 101 | | 40.4 | | | | | | | | 40.4 | | |
| | | Modified NR*81 | | 101 | | | | | | | | | | | |
| | | Modified NR · BR*82 | | | | 100.6 | | | | | | | | | |
| | | Modified NR · BR*83 | | | | | 100.6 | | | | | | | | |
| | | Modified IR*84 | | | | | | | 101 | | | | | | |
| | | Modified NBR*85 | | | | | | | | 101 | | | | 101 | 101 |
| | | S-SBR*1 | | | 82.5 | | | | | | | | 82.5 | | |
| | | NR*13 | | | | | | 40 | | | | 35 | | | |
| | | BR*12 | | | | | | 10 | | | | 15 | | | |
| | | Carbon black*20 | 4 | 4 | 4 | 4 | 4 | 4 | 4 | | 84 | | 84 | | |
| | | Carbon black*21 | | | | | | | | | | | | 50 | 50 |
| | | Carbon black*23 | | | | | | | | | | 50 | | | |
| | | Silica*27 | 80 | 80 | 80 | 80 | 80 | 80 | 80 | | | | | | |
| | | Silica*24 | | | | | | | | 50 | | | | | |
| | | Silane coupling agent*30 | 6.4 | 6.4 | 6.4 | 6.4 | 6.4 | 6.4 | 6.4 | 2.2 | | | | | |
| | | Wax*41 | 1.5 | 1.5 | 1.5 | 1.5 | 1.5 | 1.5 | 1.5 | | 1.5 | 1 | 1.5 | | |
| | | Oil*47 | 30 | 30 | 7.5 | 30 | 30 | 40 | 30 | | 7.5 | 12 | 7.5 | | |
| | | Stearic acid*42 | 2 | 2 | 2 | 2 | 2 | 2 | 2 | 1 | 2 | 1 | 2 | 1 | 1 |
| | | Zinc oxide*43 | | | 2 | | | 2 | | | | 3 | 2 | | |
| | | Antioxidant*34 | 2 | 2 | 2 | 2 | 2 | 2 | 2 | | 2 | 3 | 2 | | |
| | | Antioxidant*37 | | | | | | | | 1 | | | | 1 | 1 |
| | Step (B) | Zinc oxide*43 | 2 | 2 | | 2 | 2 | | 2 | 5 | 2 | | | 5 | 5 |
| | | Vulcanization accelerator*35 | 1 | 1 | 1 | 1 | 1 | 1 | 1 | | | | 1 | | |
| | | Vulcanization accelerator*36 | 1.5 | 1.5 | 1.5 | 1.5 | 1.5 | 1.5 | 1.5 | | 1.5 | 1.5 | 1.5 | | |
| | | Vulcanization accelerator*40 | | | | | | | | 1.5 | | | | 1.5 | |
| | | Vulcanization accelerator*38 | | | | | | | | 1.5 | | | | 1 | |
| | | Crosslinking agent*39 | | | | | | | | | | | | | 2 |
| | | Sulfur*45 | 1.5 | 1.5 | 1.5 | 1.5 | 1.5 | 1.5 | 1.5 | 1.5 | 1.5 | 1.5 | 1.5 | 1 | |

Table 11

| Components (parts by mass) | | | Example | | | | | | | | |
|---|---|---|---|---|---|---|---|---|---|---|---|
| | | | 107 | 108 | 109 | 110 | 111 | 112 | 113 | 114 | 115 |
| Components of the rubber composition | Step (A) | Modified S-SBR/BR*86 | 131.5 | | | | | | | | |
| | | Modified S-SBR/BR*87 | | 131.5 | | | | | | | |
| | | Modified S-SBR/BR*88 | | | 131.5 | | | | | | |
| | | Modified S-SBR/BR*89 | | | | 133 | | | | | |
| | | Modified S-SBR/BR*90 | | | | | 135 | | | | |
| | | Modified S-SBR/BR*74 | | | | | | 131 | 131 | 131 | |
| | | Modified CR*91 | | | | | | | | | 100 |
| | | Carbon black*20 | 4 | 4 | 4 | 4 | 4 | 4 | 4 | 4 | |
| | | Carbon black*21 | | | | | | | | | 5 |
| | | Silica*27 | 80 | 80 | 80 | 80 | 80 | 80 | 80 | 80 | |
| | | Silica*26 | | | | | | | | | 40 |
| | | Silane coupling agent*30 | 6.4 | 6.4 | 6.4 | 6.4 | 6.4 | | | | 2.4 |
| | | Silane coupling agent*31 | | | | | | 6.4 | | | |
| | | Silane coupling agent*32 | | | | | | | 6.4 | | |
| | | Silane coupling agent*33 | | | | | | | | 6.4 | |
| | | Wax*41 | 1.5 | 1.5 | 1.5 | 1.5 | 1.5 | 1.5 | 1.5 | 1.5 | |
| | | Oil*47 | 7.5 | 7.5 | 7.5 | 7.5 | 7.5 | 7.5 | 7.5 | 7.5 | 3 |
| | | Stearic acid*42 | 2 | 2 | 2 | 2 | 2 | 2 | 2 | 2 | 1.5 |
| | | Zinc oxide*43 | | | | 2 | 2 | 2 | 2 | 2 | |
| | | Antioxidant*34 | 2 | 2 | 2 | 2 | 2 | 2 | 2 | 2 | |
| | | Antioxidant*49 | | | | | | | | | 1 |
| | | Antioxidant*50 | | | | | | | | | 2 |
| | | Plasticizer*48 | | | | | | | | | 6.8 |
| | Step (B) | Zinc oxide*43 | 2 | 2 | 2 | | | | | | 5 |
| | | Magnesium oxide*44 | | | | | | | | | 4 |
| | | Vulcanization accelerator*35 | 1 | 1 | 1 | 1 | 1 | 1 | 1 | 1 | |
| | | Vulcanization accelerator*36 | 1.5 | 1.5 | 1.5 | 1.5 | 1.5 | 1.5 | 1.5 | 1.5 | |
| | | Vulcanization accelerator*38 | | | | | | | | | 0.5 |
| | | Sulfur*45 | 1.5 | 1.5 | 1.5 | 1.5 | 1.5 | 1.5 | 1.5 | 1.5 | |

Table 12

| Components (parts by mass) | | | Example | | | | | | | | | |
|---|---|---|---|---|---|---|---|---|---|---|---|---|
| | | | 116 | 117 | 118 | 119 | 120 | 121 | 122 | 123 | 124 | 125 |
| Components of the rubber composition | Step (A) | Modified S-SBR/BR*76 | 65 | 65 | | | | | | | | |
| | | Modified S-SBR/BR*92 | | | 97.5 | 97.5 | | | | | | 97.5 |
| | | Modified S-SBR/BR*74 | | | | | 130 | | | | | |
| | | Modified S-SBR/BR*93 | | | | | | 97.5 | 97.5 | | | |
| | | Modified S-SBR/BR*94 | | | | | | | | 97.5 | 97.5 | |
| | | NR*13 | 50 | 50 | 25 | 25 | | 25 | 25 | 25 | 25 | 25 |
| | | Carbon black*20 | 4 | 4 | 4 | 4 | 4 | 4 | 4 | 4 | 4 | 40 |
| | | Silica*27 | 50 | 80 | 50 | 80 | 50 | 50 | 80 | 50 | 80 | 40 |
| | | Silane coupling agent*30 | 4 | 6.4 | 4 | 6.4 | 4 | 4 | 6.4 | 4 | 6.4 | 3.2 |
| | | Wax*41 | 1.5 | 1.5 | 1.5 | 1.5 | 1.5 | 1.5 | 1.5 | 1.5 | 1.5 | 1.5 |
| | | Oil*47 | | 22.5 | | 15 | | | 15 | | 15 | 15 |
| | | Stearic acid*42 | 2 | 2 | 2 | 2 | 2 | 2 | 2 | 2 | 2 | 2 |
| | | Zinc oxide*43 | 2 | 2 | 2 | 2 | 2 | 2 | 2 | 2 | 2 | 2 |
| | | Antioxidant*34 | 2 | 2 | 2 | 2 | 2 | 2 | 2 | 2 | 2 | 2 |
| | Step (B) | Vulcanization accelerator*35 | 1 | 1 | 1 | 1 | 1 | 1 | 1 | 1 | 1 | 1 |
| | | Vulcanization accelerator*36 | 1.5 | 1.5 | 1.5 | 1.5 | 1.5 | 1.5 | 1.5 | 1.5 | 1.5 | 1.5 |
| | | Sulfur*45 | 1.5 | 1.5 | 1.5 | 1.5 | 1.5 | 1.5 | 1.5 | 1.5 | 1.5 | 1.5 |

Table 13

| Components (parts by mass) | | | Example | | | |
|---|---|---|---|---|---|---|
| | | | 126 | 127 | 128 | 129 |
| Components of the rubber composition | Step (A) | Modified S-SBR/BR*95 | 61.3 | 61.3 | | |
| | | Modified S-SBR/BR*96 | | | 91.9 | 91.9 |
| | | NR*13 | 50 | 50 | 25 | 25 |
| | | Carbon black*20 | 4 | 4 | 4 | 4 |
| | | Silica*27 | 50 | 80 | 50 | 80 |
| | | Silane coupling agent*30 | 4 | 6.4 | 4 | 6.4 |
| | | Wax*41 | 1.5 | 1.5 | 1.5 | 1.5 |
| | | Oil*47 | | 26 | | 20.6 |
| | | Stearic acid*42 | 2 | 2 | 2 | 2 |
| | | Zinc oxide*43 | 2 | 2 | 2 | 2 |
| | | Antioxidant*34 | 2 | 2 | 2 | 2 |
| | Step (B) | Vulcanization accelerator*35 | 1 | 1 | 1 | 1 |
| | | Vulcanization accelerator*36 | 1.5 | 1.5 | 1.5 | 1.5 |
| | | Sulfur*45 | 1.5 | 1.5 | 1.5 | 1.5 |

Table 14

| Components (parts by mass) | | | Example | | | |
|---|---|---|---|---|---|---|
| | | | 130 | 131 | 132 | 133 |
| Components of the rubber composition | Step (A-1) | S-SBR*1 | 110 | 110 | 110 | 110 |
| | | BR*12 | 20 | 20 | 20 | 20 |
| | | Compound (1b) *52 | 1 | 1 | 1 | 1 |
| | Step (A-2) | Carbon black*20 | 4 | 4 | 4 | 4 |
| | | Silica*27 | 80 | 80 | 80 | 80 |
| | | Silane coupling agent*30 | 6.4 | 6.4 | 6.4 | 6.4 |
| | | Wax*41 | 1.5 | 1.5 | 1.5 | 1.5 |
| | | Oil*47 | 7.5 | 7.5 | 7.5 | 7.5 |
| | | Stearic acid*42 | 2 | 2 | 2 | 2 |
| | | Zinc oxide*43 | 2 | 2 | 2 | 2 |
| | | Antioxidant*34 | 2 | 2 | 2 | 2 |
| | Step (B) | Vulcanization accelerator*35 | 1 | 1 | 1 | 1 |
| | | Vulcanization accelerator*36 | 1.5 | 1.5 | 1.5 | 1.5 |
| | | Sulfur*45 | 1.5 | 1.5 | 1.5 | 1.5 |
| Step (A-1): Mixture temperature (°C) | | | 100 | 130 | 140 | 150 |
| Step (A-1): Kneading time (second) | | | 300 | 300 | 100 | 300 |

Low heat build-up (tan δ index) test

The tan δ of the rubber compositions obtained in the following Examples 1 to 133 was measured using a viscoelasticity measuring instrument (produced by Metravib) at a temperature of 40°C, a dynamic strain of 5%, and a frequency of 15 Hz. For comparison, rubber compositions (reference compositions) were prepared using the same formulations and the same production methods as in each of the Examples except that no tetrazine compound was added. The inverse of the tan δ of each reference rubber composition was defined as 100. The low heat build-up index was calculated according to the following formula. A higher low heat build-up index indicates a lower heat build-up and a smaller hysteresis loss. The low heat build-up of each reference vulcanized rubber composition was defined as 100.
Formula:

Low heat build-up index = {(tan δ of the rubber composition not containing the tetrazine compound (1))/(tan δ of the rubber composition of the present invention)} x 100

All the rubber compositions obtained in the Examples exhibited excellent resistance to heat build-up, as compared with the comparative rubber compositions containing no tetrazine compound. Among these, the rubber compositions obtained in Examples 2, 4, 7, 11, 21, 22, 24, 26, 27, 36, 37, 41, 42, 52 to 54, 73, 75, 76, 81, 84, 88, 122, 123, and 131 to 133 exhibited a low heat build-up index of 130 or more and less than 140, and the rubber compositions obtained in Examples 13, 29, 63, 68, 72, 74, 83, 85, 87, 111, 113, 119, 124, and 128 exhibited a low heat build-up index of 140 or more and less than 150. Further, the rubber compositions obtained in Examples 3, 5, 19, 30, 35, 62, 66, 67, 79, 82, 99, 110, 114, 116, 117, 126, 127, and 129 exhibited a low heat build-up index of 150 or more.

Industrial Applicability

The rubber composition used in the present invention, which contains a tetrazine compound (1), has enhanced dispersibility of inorganic fillers (e.g., silica) and/or carbon black, and has excellent low heat build-up. The rubber composition used in the present invention has excellent low heat build-up, even when no silane coupling agent is incorporated in the rubber composition. Accordingly, the rubber composition of the present invention can be used for tread, sidewall, bead area, belt, carcass and shoulder portions of various types of pneumatic tires for various vehicles, especially for tread, sidewall, bead area, belt, carcass, and shoulder portions of pneumatic radial tires.

## Claims

1. Use of an additive for imparting low heat build-up to a rubber component for at least one member selected from tread, sidewall, bead area, belt, carcass, and shoulder portions of a tire, the additive comprising

   a tetrazine compound represented by general formula (1):

   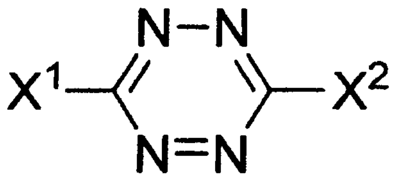

   (1)

   wherein $X^1$ and $X^2$ are the same or different and represent a hydrogen atom or an alkyl, alkylthio, aralkyl, aryl, arylthio, heterocyclic, or amino group; and each of these groups may have one or more substituents, or
   a salt thereof.

2. The use according to claim 1, wherein $X^1$ and $X^2$ represent a heterocyclic group.

3. The use according to claim 1 or 2, wherein the rubber component is a diene rubber.

4. Use of a modified polymer produced using a rubber mixture comprising a rubber component and an additive for imparting low heat build-up to the rubber component for at least one member selected from tread, sidewall, bead area, belt, carcass and shoulder portions of a tire,the additive comprising a tetrazine compound represented by general formula (1):

   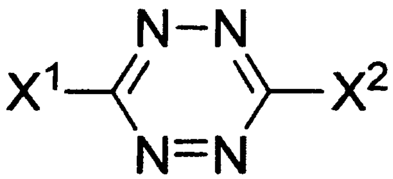

   (1)

   wherein $X^1$ and $X^2$ are the same or different and represent a hydrogen atom or an alkyl, alkylthio, aralkyl, aryl, arylthio, heterocyclic, or amino group; and each of these groups may have one or more substituents, or
   a salt thereof.

5. The use according to claim 4, wherein the rubber component is a diene rubber.

6. Use of a rubber composition comprising a rubber component, an additive for imparting low heat build-up to the rubber component, and an inorganic filler and/or carbon black for at least one member selected from tread, sidewall, bead area, belt, carcass and shoulder portions of a tire,

   the additive comprising
   a tetrazine compound represented by general formula (1):

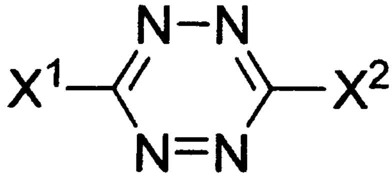

(1)

wherein $X^1$ and $X^2$ are the same or different and represent a hydrogen atom or an alkyl, alkylthio, aralkyl, aryl, arylthio, heterocyclic, or amino group; and each of these groups may have one or more substituents, or a salt thereof.

**7.** Use of a rubber composition comprising a modified polymer produced using a rubber mixture comprising a rubber component and an additive for imparting low heat build-up to the rubber component; and an inorganic filler and/or carbon black for at least one member selected from tread, sidewall, bead area, belt, carcass, and shoulder portions of a tire,

the additive comprising
a tetrazine compound represented by general formula (1):

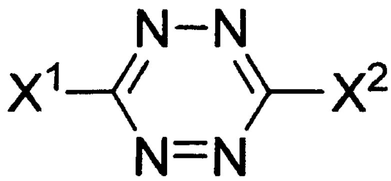

(1)

wherein $X^1$ and $X^2$ are the same or different and represent a hydrogen atom or an alkyl, alkylthio, aralkyl, aryl, arylthio, heterocyclic, or amino group; and each of these groups may have one or more substituents, or a salt thereof.

**8.** The use according to claim 6, wherein the rubber component is a diene rubber.

**9.** A tread, sidewall, bead area, belt, carcass, or shoulder portion of a tire produced using a rubber composition comprising a modified polymer produced using a rubber mixture comprising a rubber component and an additive for imparting low heat build-up to the rubber component; and an inorganic filler and/or carbon black,

the additive comprising
a tetrazine compound represented by general formula (1):

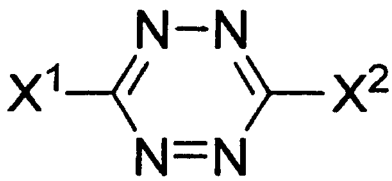

(1)

wherein $X^1$ and $X^2$ are the same or different and represent a hydrogen atom or an alkyl, alkylthio, aralkyl, aryl, arylthio, heterocyclic, or amino group; and each of these groups may have one or more substituents, or a salt thereof.

**10.** A tire having at least one member selected from a group consisting of tread, sidewall, bead area, belt, carcass, and shoulder portions according to claim 9.

## Patentansprüche

1. Verwendung eines Additivs zur Verleihung eines geringen Wärmeaufbaus an einer Kautschukkomponente für mindestens ein Element, ausgewählt aus Profil, Seitenwand, Wulstbereich, Gürtel, Karkasse und Schulterabschnitten eines Reifens, wobei das Additiv eine Tetrazinverbindung, dargestellt durch die allgemeine Formel (1) :

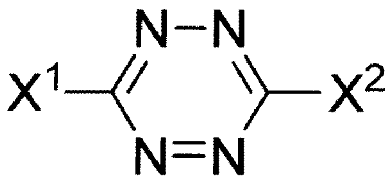

(1)

wobei $X^1$ und $X^2$ gleich oder verschieden sind und ein Wasserstoffatom oder eine Alkyl-, Alkylthio-, Aralkyl-, Aryl-, Arylthio-, heterocyclische oder Aminogruppe darstellen; und jede dieser Gruppen einen oder mehrere Substituenten aufweisen kann oder
ein Salz davon umfasst.

2. Die Verwendung gemäß Anspruch 1, wobei $X^1$ und $X^2$ eine heterocyclische Gruppe darstellen.

3. Die Verwendung gemäß Anspruch 1 oder 2, wobei die Kautschukkomponente ein Dienkautschuk ist.

4. Verwendung eines modifizierten Polymers, hergestellt unter Verwendung eines Kautschukgemisches, umfassend eine Kautschukkomponente und ein Additiv zur Verleihung eines geringen Wärmeaufbaus an der Kautschukkomponente für mindestens ein Element, ausgewählt aus Profil, Seitenwand, Wulstbereich, Gürtel, Karkasse und Schulterabschnitten eines Reifens, wobei das Additiv eine Tetrazinverbindung, dargestellt durch die allgemeine Formel (1) :

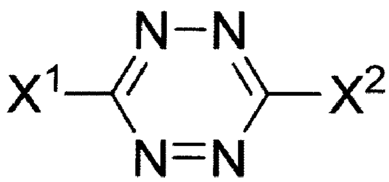

(1)

wobei $X^1$ und $X^2$ gleich oder verschieden sind und ein Wasserstoffatom oder eine Alkyl-, Alkylthio-, Aralkyl-, Aryl-, Arylthio-, heterocyclische oder Aminogruppe darstellen; und jede dieser Gruppen einen oder mehrere Substituenten aufweisen kann oder
ein Salz davon umfasst.

5. Die Verwendung gemäß Anspruch 4, wobei die Kautschukkomponente ein Dienkautschuk ist.

6. Verwendung einer Kautschukzusammensetzung, umfassend eine Kautschukkomponente, ein Additiv zur Verleihung eines geringen Wärmeaufbaus an der Kautschukkomponente und einen anorganischen Füllstoff und/oder Carbon Black, für mindestens ein Element, ausgewählt aus Profil, Seitenwand, Wulstbereich, Gürtel, Karkasse und Schulterabschnitten eines Reifens,

wobei das Additiv
eine Tetrazinverbindung, dargestellt durch die allgemeine Formel (1) :

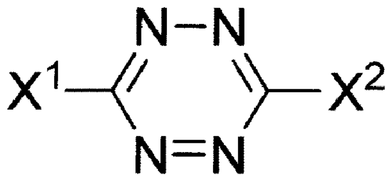

(1)

wobei $X^1$ und $X^2$ gleich oder verschieden sind und ein Wasserstoffatom oder eine Alkyl-, Alkylthio-, Aralkyl-, Aryl-, Arylthio-, heterocyclische oder Aminogruppe darstellen; und jede dieser Gruppen einen oder mehrere Substituenten aufweisen kann oder
ein Salz davon umfasst.

**7.** Verwendung einer Kautschukzusammensetzung, umfassend ein modifiziertes Polymer, hergestellt unter Verwendung eines Kautschukgemisches, umfassend eine Kautschukkomponente und ein Additiv zur Verleihung eines geringen Wärmeaufbaus an der Kautschukkomponente; und einen anorganischen Füllstoff und/oder Carbon Black, für mindestens ein Element, ausgewählt aus Profil, Seitenwand, Wulstbereich, Gürtel, Karkasse und Schulterabschnitten eines Reifens,

wobei das Additiv
eine Tetrazinverbindung, dargestellt durch die allgemeine Formel (1) :

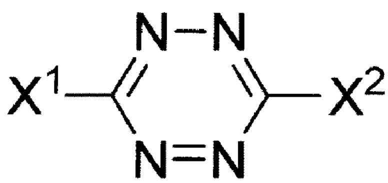

(1)

wobei $X^1$ und $X^2$ gleich oder verschieden sind und ein Wasserstoffatom oder eine Alkyl-, Alkylthio-, Aralkyl-, Aryl-, Arylthio-, heterocyclische oder Aminogruppe darstellen; und jede dieser Gruppen einen oder mehrere Substituenten aufweisen kann oder
ein Salz davon umfasst.

**8.** Die Verwendung gemäß Anspruch 6, wobei die Kautschukkomponente ein Dienkautschuk ist.

**9.** Ein(e) Profil, Seitenwand, Wulstbereich, Gürtel, Karkasse oder Schulterabschnitt eines Reifens, hergestellt unter Verwendung einer Kautschukzusammensetzung, umfassend ein modifiziertes Polymer, hergestellt unter Verwendung eines Kautschukgemisches, umfassend eine Kautschukkomponente und ein Additiv zur Verleihung eines geringen Wärmeaufbaus an der Kautschukkomponente; und einen anorganischen Füllstoff und/oder Carbon Black,

wobei das Additiv
eine Tetrazinverbindung, dargestellt durch die allgemeine Formel (1) :

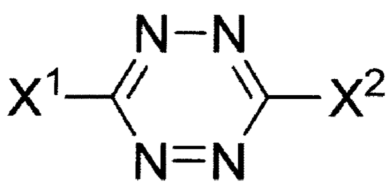

(1)

wobei $X^1$ und $X^2$ gleich oder verschieden sind und ein Wasserstoffatom oder eine Alkyl-, Alkylthio-, Aralkyl-, Aryl-, Arylthio-, heterocyclische oder Aminogruppe darstellen; und jede dieser Gruppen einen oder mehrere Substituenten aufweisen kann oder
ein Salz davon umfasst.

10. Ein Reifen mit mindestens einem Element, ausgewählt aus einer Gruppe bestehend aus Profil, Seitenwand, Wulstbereich, Gürtel, Karkasse und Schulterabschnitten gemäß Anspruch 9.

## Revendications

1. Utilisation d'un additif pour conférer une faible accumulation de chaleur à un composant de caoutchouc pour au moins un élément choisi parmi la bande de roulement, le flanc, la zone de talon, la ceinture, la carcasse, et des parties d'épaulement d'un pneu, l'additif comprenant

   un composé tétrazine représenté par la formule générale (1) :

   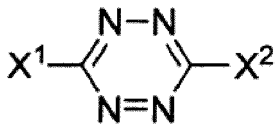

   (1)

   dans laquelle $X^1$ et $X^2$ sont identiques ou différents et représentent un atome d'hydrogène ou un groupe alkyle, alkylthio, aralkyle, aryle, arylthio, hétérocyclique, ou amino ; et chacun de ces groupes peut avoir un ou plusieurs substituants, ou
   un sel de celui-ci.

2. Utilisation selon la revendication 1, dans laquelle $X^1$ et $X^2$ représentent un groupe hétérocyclique.

3. Utilisation selon la revendication 1 ou 2, dans laquelle le composant de caoutchouc est un caoutchouc de diène.

4. Utilisation d'un polymère modifié produit en utilisant un mélange de caoutchouc, comprenant un composant de caoutchouc et un additif pour conférer une faible accumulation de chaleur à un composant de caoutchouc pour au moins un élément choisi parmi la bande de roulement, le flanc, la zone de talon, la ceinture, la carcasse, et des parties d'épaulement d'un pneu, l'additif comprenant

   un composé tétrazine représenté par la formule générale (1) :

   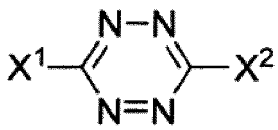

   (1)

   dans laquelle $X^1$ et $X^2$ sont identiques ou différents et représentent un atome d'hydrogène ou un groupe alkyle, alkylthio, aralkyle, aryle, arylthio, hétérocyclique, ou amino ; et chacun de ces groupes peut avoir un ou plusieurs substituants, ou
   un sel de celui-ci.

5. Utilisation selon la revendication 4, dans laquelle le composant de caoutchouc est un caoutchouc de diène.

6. Utilisation d'une composition de caoutchouc comprenant un composant de caoutchouc, un additif pour conférer une faible accumulation de chaleur au composant de caoutchouc, et une charge inorganique et/ou du noir de carbone pour au moins un élément choisi parmi la bande de roulement, le flanc, la zone de talon, la ceinture, la carcasse, et des parties d'épaulement d'un pneu,

   l'additif comprenant
   un composé tétrazine représenté par la formule générale (1) :

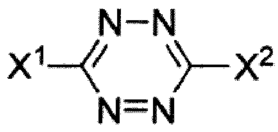

(1)

dans laquelle $X^1$ et $X^2$ sont identiques ou différents et représentent un atome d'hydrogène ou un groupe alkyle, alkylthio, aralkyle, aryle, arylthio, hétérocyclique, ou amino ; et chacun de ces groupes peut avoir un ou plusieurs substituants, ou
un sel de celui-ci.

**7.** Utilisation d'une composition de caoutchouc comprenant un polymère modifié produit en utilisant un mélange de caoutchouc comprenant un composant de caoutchouc et un additif pour conférer une faible accumulation de chaleur au composant de caoutchouc ; et une charge inorganique et/ou du noir de carbone pour au moins un élément choisi parmi la bande de roulement, le flanc, la zone de talon, la ceinture, la carcasse, et des parties d'épaulement d'un pneu,

l'additif comprenant
un composé tétrazine représenté par la formule générale (1) :

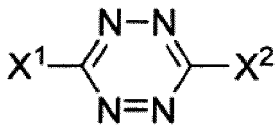

(1)

dans laquelle $X^1$ et $X^2$ sont identiques ou différents et représentent un atome d'hydrogène ou un groupe alkyle, alkylthio, aralkyle, aryle, arylthio, hétérocyclique, ou amino ; et chacun de ces groupes peut avoir un ou plusieurs substituants, ou
un sel de celui-ci.

**8.** Utilisation selon la revendication 6, dans laquelle le composant de caoutchouc est un caoutchouc de diène.

**9.** Bande de roulement, flanc, zone de talon, ceinture, carcasse, ou partie d'épaulement d'un pneu, produits en utilisant une composition de caoutchouc comprenant un polymère modifié produit en utilisant un mélange de caoutchouc, comprenant un composant de caoutchouc et un additif pour conférer une faible accumulation de chaleur au composant de caoutchouc ; et une charge inorganique et/ou du noir de carbone,

l'additif comprenant
un composé tétrazine représenté par la formule générale (1) :

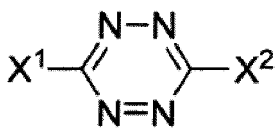

(1)

dans laquelle $X^1$ et $X^2$ sont identiques ou différents et représentent un atome d'hydrogène ou un groupe alkyle, alkylthio, aralkyle, aryle, arylthio, hétérocyclique, ou amino ; et chacun de ces groupes peut avoir un ou plusieurs substituants, ou
un sel de celui-ci.

**10.** Pneu ayant au moins un élément choisi dans un groupe consistant en bande de roulement, flanc, zone de talon, ceinture, carcasse, et parties d'épaulement selon la revendication 9.

Fig. 1

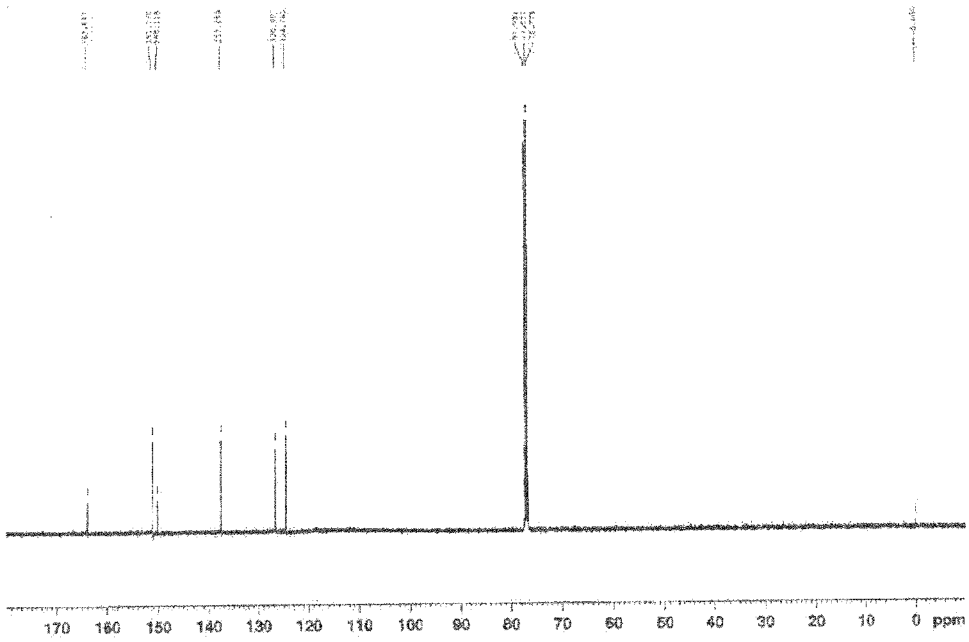

Fig. 2

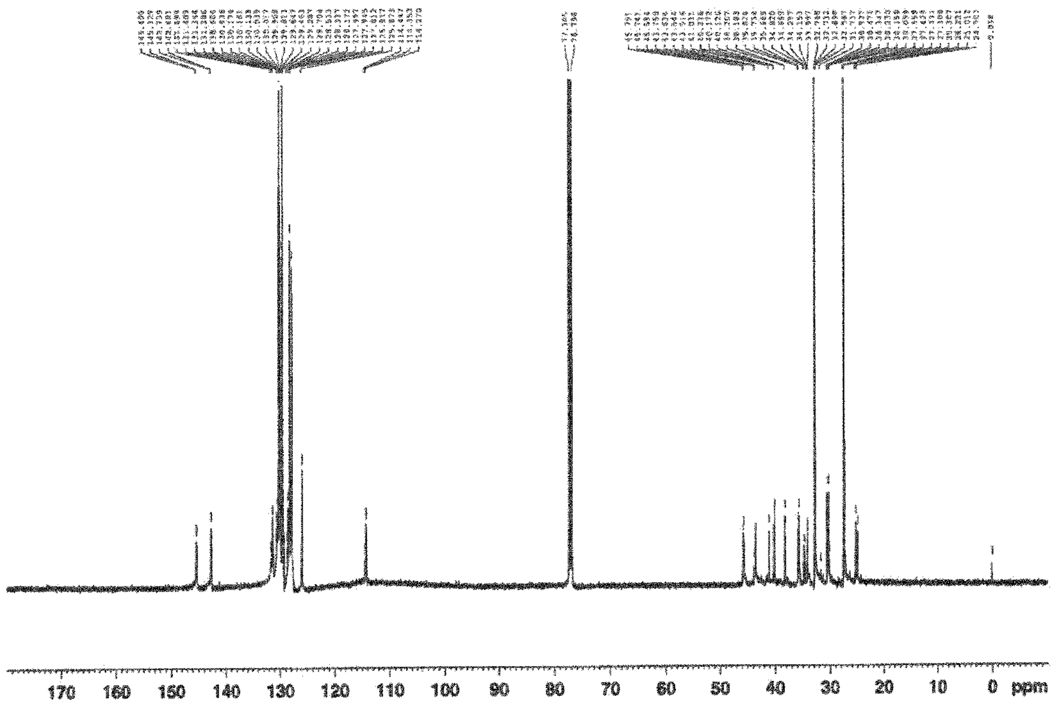

Fig. 3

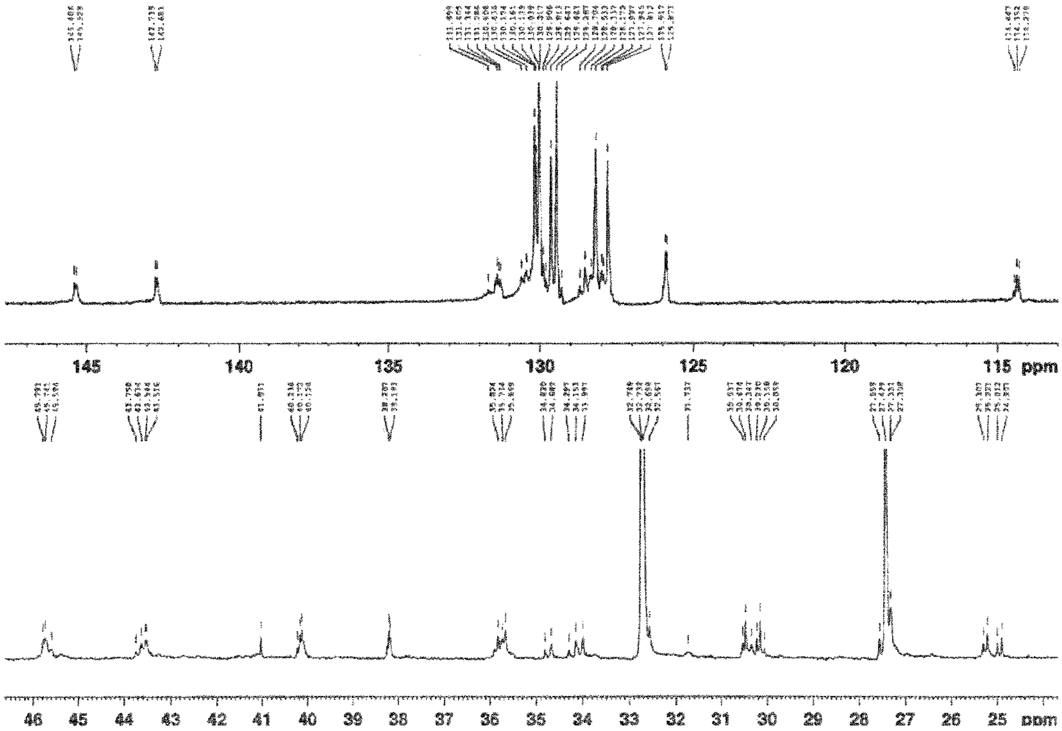


Fig. 4

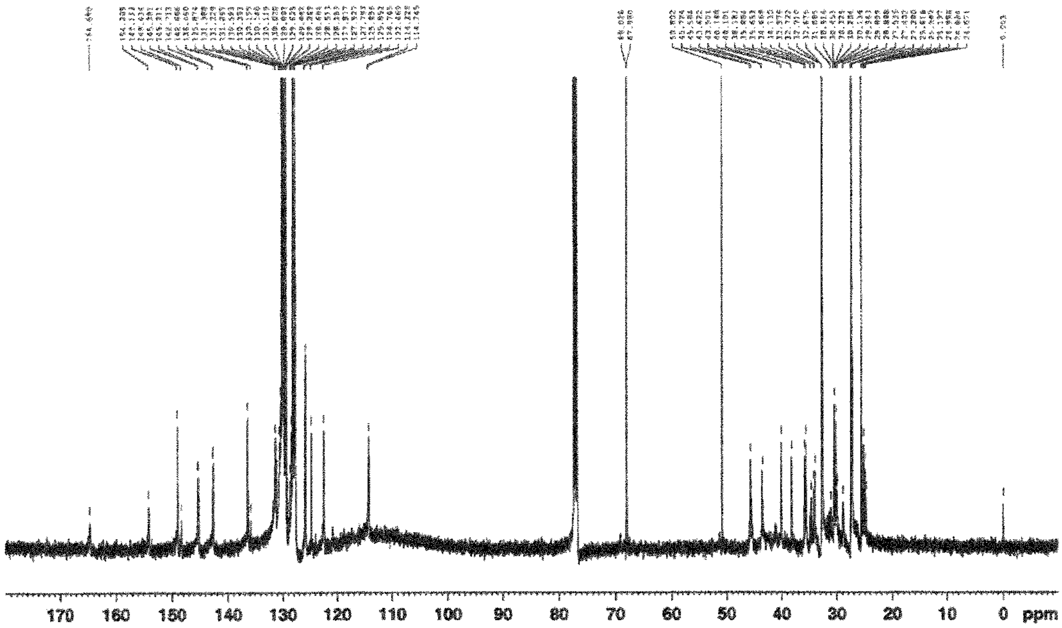


The peaks at 68 ppm and 51 ppm are derived from THF used in the extraction.

Fig. 5

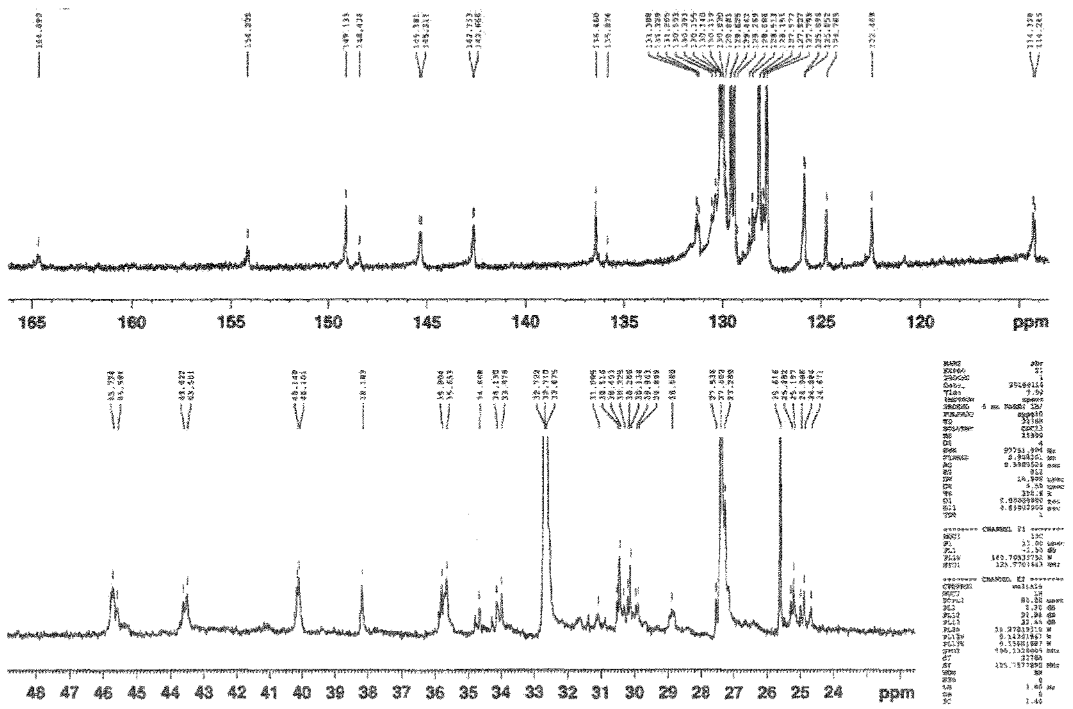


Fig. 6

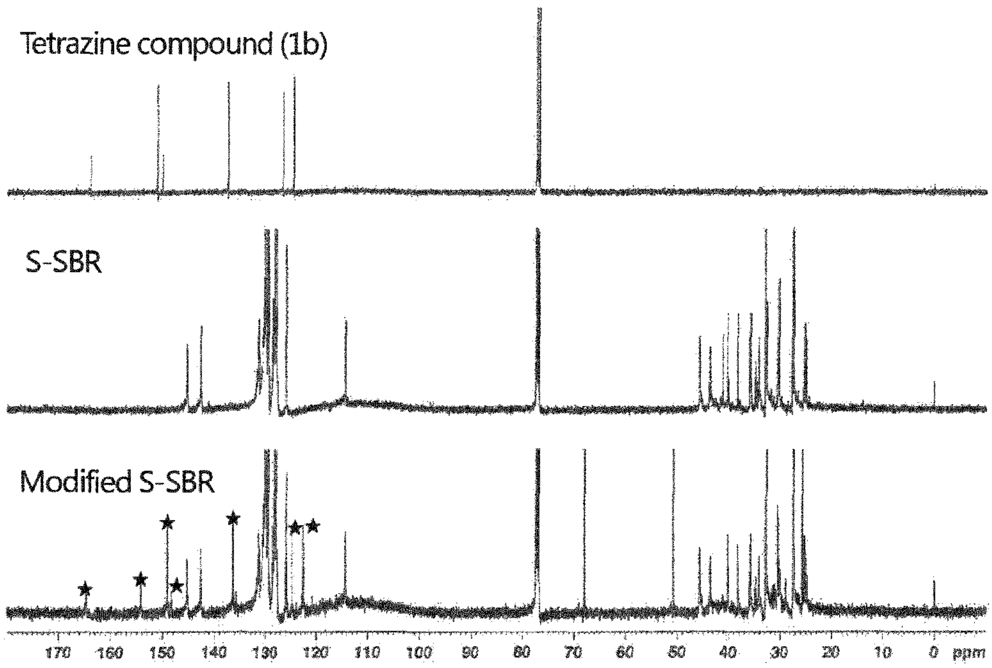


★: Newly confirmed peaks

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2003514079 A
- JP 2003523472 A
- JP 2013108004 A
- JP 2000169631 A
- JP 2005220323 A
- JP 2015093928 A
- US 20140113844 A1